(19) Europäisches Patentamt / European Patent Office / Office européen des brevets

(11) **EP 4 159 763 A1**

(12) **EUROPEAN PATENT APPLICATION**
published in accordance with Art. 153(4) EPC

(43) Date of publication:
**05.04.2023 Bulletin 2023/14**

(21) Application number: **21812743.9**

(22) Date of filing: **28.05.2021**

(51) International Patent Classification (IPC):
**C07K 16/30** (2006.01)   **A61K 39/395** (2006.01)
**A61K 45/00** (2006.01)   **A61K 47/68** (2017.01)
**A61P 35/00** (2006.01)   **A61P 43/00** (2006.01)
**C07K 16/46** (2006.01)   **C12Q 1/04** (2006.01)

(52) Cooperative Patent Classification (CPC):
**A61K 39/395; A61K 45/00; A61K 47/68;**
**A61P 35/00; A61P 43/00; C07K 16/30;**
**C07K 16/46; C12Q 1/04**

(86) International application number:
**PCT/JP2021/020384**

(87) International publication number:
**WO 2021/241729 (02.12.2021 Gazette 2021/48)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **29.05.2020 JP 2020094545**

(71) Applicant: **BrightPath Biotherapeutics Co., Ltd.**
**Kawasaki-shi, Kanagawa 210-0821 (JP)**

(72) Inventors:
• **NAKAMURA, Norihiro**
  **Kawasaki-shi, Kanagawa 210-0821 (JP)**
• **NOZAWA, Risa**
  **Kawasaki-shi, Kanagawa Prefecture 211-0012**
  **(JP)**

• **OBONAI, Toshifumi**
  **Kawasaki-shi, Kanagawa 210-0821 (JP)**
• **BAN, Haruka**
  **Kawasaki-shi, Kanagawa 210-0821 (JP)**
• **SASAKURA, Yukie**
  **Yokohama-shi, Kanagawa 230-0052 (JP)**
• **MISHIMA, Yuji**
  **Kawasaki-shi, Kanagawa 210-0821 (JP)**
• **MATSUMOTO, Noriko**
  **Kawasaki-shi, Kanagawa 210-0821 (JP)**

(74) Representative: **Plasseraud IP**
  **66, rue de la Chaussée d'Antin**
  **75440 Paris Cedex 09 (FR)**

Remarks:
The complete document including Reference
Table(s) and the Sequence Listing(s) can be
downloaded from the EPO website

(54) **ANTI-CD73 ANTIBODY AND USE THEREOF**

(57)   A problem to be solved by the present invention is to develop an antibody having a higher function targeting a CD73 antigen, and more specifically, an antibody for cancer treatment having a higher activity. The present invention has shown that the above problem can be solved by finding an antibody or a human-type antibody derivative that has a binding activity to a CD73 antigen and activates T cells having a toxic activity to cancer cells, comprising complementarity determining regions of any combination of heavy and light chains each having a specific amino acid sequence.

**Description**

Technical Field

The present invention relates to an antibody having cytotoxic activity specifically on a cancer expressing a CD73 antigen, and a composition and a method for cancer treatment and for cancer screening comprising the antibody.

Background Art

CD73 is an enzyme that catalyzes the reaction that degrades AMP into adenosine in vivo, and is known to be expressed on the cell surface of cancer cells, regulatory T cells (Treg cells), and vascular endothelial cells. It is known that the adenosine generated by CD73 on the surface of these cells is released into a microenvironment, binds to an adenosine receptor on immune cells, and induces exhaustion of the immune cells.

It has been reported that the CD73 antigen is highly expressed in a plurality of carcinomas, and that cases having high expression of the CD73 antigen show poor prognosis. It is thought that the poor prognosis occurs because the increase in adenosine in the microenvironment around cancer cells suppresses antitumor immunity and because the released adenosine promotes the suppression of immune activity by Treg cells.

Meanwhile, it has been reported that, in a non-clinical model, an antibody against the CD73 antigen has a tumor proliferation suppressive effect (Oncoimmunology. 2016 Aug; 5(8): e1208875). Based on these findings, clinical development (Phases I to II) of antibodies for cancer treatment targeting the CD73 antigen is underway. However, no antibodies has been led to start of a confirmatory clinical trial, and there still be a need for the development of an antibody having a higher function.

Citation List

Patent Literature

Patent Literature 1: Japanese Patent Public Disclosure No. 2018-501197

Non Patent Literature

Non Patent Literature 1: Oncoimmunology. 2016 Aug; 5(8): e1208875
Non Patent Literature 2: Bioinformatics. 2015 Feb 1;31(3):434-5

Summary of Invention

Technical Problem

A problem to be solved by the present invention is to develop an antibody having a higher function targeting a CD73 antigen, and more specifically, a therapeutic antibody for cancer treatment having a higher activity.

Solution to Problem

The present invention has shown that the above problem can be solved by finding an antibody or a human-type antibody derivative that has a binding activity to a CD73 antigen and activates T cells having the toxic activity to cancer cells, comprising complementarity determining regions of any combination of heavy and light chains each having a designated amino acid sequence. Specifically, the antibody is a monoclonal antibody that specifically binds to the CD73 antigen and has the following functions:

- showing the effect of more strongly suppressing the reaction that degrades AMP into adenosine by CD73 than a benchmark antibody (antibody having the same sequences as those of the published variable regions of MEDI9447); and
- releasing adenosine-induced exhaustion of T cells to activate the T cells.

More specifically, the present application provides the following aspects in order to solve the above problem:

[1]: An antibody or a human-type antibody derivative that has a binding activity to a CD73 antigen and activates T cells having a toxic activity to cancer cells, comprising complementarity determining regions of any combination of heavy and light chains selected from the group consisting of

(1) complementarity determining regions of a heavy chain, CDR1 (DX$_1$NMD (wherein X$_1$ is C or S), SEQ ID No.: 1), CDR2 (DINPNNGGTIYNQKFKG, SEQ ID No.: 2), and CDR3 (TNWDYAMDY, SEQ ID No.: 3) and complementarity determining regions of a light chain, CDR1 (KASQDINSX$_2$LS (wherein X$_2$ is N, D, or Q), SEQ ID No.: 4), CDR2 (RANRLID, SEQ ID No.: 5), and CDR3 (X$_3$QYDVFPRT (wherein X$_3$ is L or Q), SEQ ID No.: 6);

(2) complementarity determining regions of a heavy chain, CDR1 (SFGMH, SEQ ID No.: 9), CDR2 (YISSGSRTIYYADTVRG, SEQ ID No.: 10), and CDR3 (DFGSSSPNYFDY, SEQ ID No.: 11), and complementarity determining regions of a light chain, CDR1 (RASESVDNYGISFMN, SEQ ID No.: 12), CDR2 (AASNQGS, SEQ ID No.: 13), and CDR3 (QQSKEVPWT, SEQ ID No.: 14);

(3) complementarity determining regions of a heavy chain, CDR1 (GYWMN, SEQ ID No.: 17), CDR2 (RIDPYDSETHYSQKFKD, SEQ ID No.: 18), and CDR3 (SSPITTAPFDY, SEQ ID No.: 19), and complementarity determining regions of a light chain, CDR1 (RASESVDYYGFSFMN, SEQ ID No.: 20), CDR2 (AASTQGS, SEQ ID No.: 21), and CDR3 (QQSKEVPYT, SEQ ID No.: 22);

(4) complementarity determining regions of a heavy chain, CDR1 (SYGVS, SEQ ID No.: 25), CDR2 (VIWGDGSTNYHSALIS, SEQ ID No.: 26), and CDR3 (TNIFYDYDWYLDV, SEQ ID No.: 27), and complementarity determining regions of a light chain, CDR1 (RSSQSLVHSNGNTYLH, SEQ ID No.: 28), CDR2 (KVSNRFS, SEQ ID No.: 29), and CDR3 (SHSTHVPWT, SEQ ID No.: 30);

(5) complementarity determining regions of a heavy chain, CDR1 (SYWMH, SEQ ID No.: 33), CDR2 (EINPSNARTNYNENFKS, SEQ ID No.: 34), and CDR3 (RGTSGNYFDF, SEQ ID No.: 35), and complementarity determining regions of a light chain, CDR1 (KASQDINTYLS, SEQ ID No.: 36), CDR2 (RANRLVD, SEQ ID No.: 37), and CDR3 (LQYDEFPYT, SEQ ID No.: 38);

(6) complementarity determining regions of a heavy chain, CDR1 (SYWMN, SEQ ID No.: 41), CDR2 (KIDPYDSETHYNQKFKD, SEQ ID No.: 42), and CDR3 (IRYGTFDY, SEQ ID No.: 43), and complementarity determining regions of a light chain, CDR1 (KASQDINNYLS, SEQ ID No.: 44), CDR2 (RANILVD, SEQ ID No.: 45), and CDR3 (LQYDEFPYT, SEQ ID No.: 46);

(7) complementarity determining regions of a heavy chain, CDR1 (SYWMH, SEQ ID No.: 49), CDR2 (EINPSNGRTNYNEKFKN, SEQ ID No.: 50), and CDR3 (RGTSGNYFDY, SEQ ID No.: 51), and complementarity determining regions of a light chain, CDR1 (KASQDINTYLS, SEQ ID No.: 52), CDR2 (RANRLVD, SEQ ID No.: 53), and CDR3 (LQYDEFPYT, SEQ ID No.: 54); and

(8) complementarity determining regions of a heavy chain, CDR1 (SYWMN, SEQ ID No.: 57), CDR2 (RIDPYDSEAHYNQKFKD, SEQ ID No.: 58), and CDR3 (IRYGTFDY, SEQ ID No.: 59), and complementarity determining regions of a light chain, CDR1 (KASQDINSYLS, SEQ ID No.: 60), CDR2 (RSNSLVD, SEQ ID No.: 61), and CDR3 (LQYDEFPYT, SEQ ID No.: 62);

[2]: The antibody or human-type antibody derivative according to [1], wherein the activation of T cells is selected from the group consisting of T cell proliferation, increased T cell cytotoxicity against cancer cells, and promotion of T cell cytokine secretion;

[3]: The antibody or human-type antibody derivative according to [1] or [2], wherein the human-type antibody derivative is selected from a human-type antibody variant selected from a humanized antibody, a chimeric antibody, a polyvalent antibody, and a multispecific antibody, or a functional fragment thereof;

[4]: The antibody or human-type antibody derivative according to any one of [1] to [3], wherein the functional fragment is F(ab')2;

[5]: The antibody or human-type antibody derivative according to any one of [1] to [4], wherein an amino acid sequence of a heavy chain variable region VH domain of the antibody or human-type antibody derivative is selected from (1) SEQ ID No.: 7, (2) SEQ ID No.: 15, (3) SEQ ID No.: 23, (4) SEQ ID No.: 31, (5) SEQ ID No.: 39, (6) SEQ ID No.: 47, (7) SEQ ID No.: 55, and (8) SEQ ID No. 63;

[6]: The antibody or human-type antibody derivative according to any one of [1] to [5], wherein an amino acid sequence of a light chain variable region VL domain of the antibody or human-type antibody derivative is selected from (1) SEQ ID No.: 8, (2) SEQ ID No.: 16, (3) SEQ ID No.: 24, (4) SEQ ID No.: 32, (5) SEQ ID No.: 40, (6) SEQ ID No.: 48, (7) SEQ ID No.: 56, and (8) SEQ ID No. 64;

[7]: The antibody or human-type antibody derivative according to any one of [1] to [6], wherein the antibody or human-type antibody derivative induces cytotoxicity against cancer cells but does not induce cytotoxicity against normal cells;

[8]: The antibody or human-type antibody derivative according to any one of [1] to [7], wherein the cancer cells are selected from the group consisting of melanoma, breast cancer, lung cancer, and colorectal cancer;

[9]: The antibody or human-type antibody derivative according to any one of [1] to [8], wherein the antibody or human-type antibody derivative is bound to a drug to form an antibody drug conjugate (ADC) ;

[10]: A pharmaceutical composition for treating a cancer, comprising the antibody or human-type antibody derivative according to any one of [1] to [9];

[11]: The pharmaceutical composition according to [10], wherein the cancer is selected from the group consisting of melanoma, breast cancer, lung cancer, and colorectal cancer;

[12]: A method for measuring cytotoxicity against cancer cells, comprising:

a step of contacting in vitro cancer cells collected from a subject with the antibody or human-type antibody derivative according to any one of [1] to [9]; and

a step of measuring whether or not AMP metabolism of the cancer cell is reduced, a step of measuring whether or not cell viability is reduced, or a step of measuring whether or not secretion of an immunostimulatory substance is enhanced, under a culture condition;

[13]: The method for measuring cytotoxicity against cancer cells according to [12], comprising measuring, in the presence of peripheral blood lymphocytes of the same subject, whether or not cell viability of the cancer cells is reduced or whether or not immune cells derived from the peripheral blood lymphocytes are activated;

[14]: The method for measuring cytotoxicity against cancer cells according to [12] or [13], comprising measuring, from in vitro AMP metabolism suppression or cytotoxicity against the cancer cells collected from the subject, an enhancement of cytotoxicity against the cancer cells when the antibody or human-type antibody derivative according to any one of [1] to [9] is administered to the subject;

[15]: The method for measuring cytotoxicity against cancer cells according to [12] or [13], comprising measuring, from in vitro enhancement of secretion of the immunostimulatory substance, an enhancement of cytotoxicity against the cancer cells when the antibody or human-type antibody derivative according to any one of [1] to [9] is administered to the subject;

[16]: A measurement kit comprising the antibody or the human-type antibody derivative according to any one of [1] to [9] for in vitro measuring AMP metabolism, cytotoxicity, secretion of an immunostimulatory substance, or activation of immune cells against the cancer cells collected from the subject by the antibody or the human-type antibody derivative.

Advantageous Effects of Invention

The antibody or human-type antibody derivative obtained by the present invention suppresses the function of CD73 expressed in cancer cells or immune cells and releases the exhaustion of the immune cells (particularly T cells) and thereby activates the immune cells and exerts a tumor proliferation suppressive effect and a cancer treatment effect.

The antibodies of the present invention can be widely used as a therapeutic and/or diagnostic agent for cancer. The antibody according to the present invention has the effect of releasing the exhaustion of immune cells, and thus is expected to be particularly effective in a combination therapy with another immunotherapy.

Brief Description of Drawings

[Figure 1] Figure 1 is a diagram showing the AMP degradation suppressive function (H322 cell) of a 002_m antibody prepared in Example 1.

[Figure 2] Figure 2 is a diagram showing amino acid sequences of the heavy chain variable region and the light chain variable region of the 002_m antibody.

[Figure 3] Figure 3 is diagrams showing the binding activity of the obtained chimeric antibodies 002_1_c to 002_6_c to a human lung cancer cell line H322 cells expressing CD73.

[Figure 4] Figure 4 is a diagram showing the AMP degradation suppressive function using a human lung cancer H322 cells for the 002_1_c antibody to the 002_6_c antibody.

[Figure 5-1] Figure 5-1 is a diagram showing amino acid sequences of the heavy chain variable regions of the obtained humanized antibodies (002_6_h1 to 002_6_h8) of 002_6_c.

[Figure 5-2] Figure 5-2 is a diagram showing amino acid sequences of the light chain variable regions of the obtained humanized antibodies (002_6_h1 to 002_6_h8) of 002_6_c.

[Figure 6-1] Figure 6-1 is diagrams showing the binding activity of the humanized antibodies (002_6_h1 to 002_6_h8) to a human lung cancer cell line H322 cells expressing CD73.

[Figure 6-2] Figure 6-2 is diagrams showing the binding activity of the humanized antibodies (002_6_h1 to 002_6_h8) to a human lung cancer cell line H322 cells expressing CD73.

[Figure 7] Figure 7 is a diagram showing the AMP degradation suppressive function of three humanized antibodies (002_6_h2, 002_6_h4, and 002_6_h8) using a human lung cancer H322 cells.

[Figure 8-1] Figure 8-1 is diagrams showing the binding activity of the humanized antibody 002_6_h4 to various cells in comparison with an existing antibody and the base chimeric antibody 002_6_c.

[Figure 8-2] Figure 8-2 is diagrams showing the binding activity of the humanized antibody 002_6_h4 to various cells in comparison with the existing antibody and the base chimeric antibody 002_6_c.

[Figure 9] Figure 9 is a diagram showing the AMP degradation suppressive function of the humanized antibody 002_6_h4 using a human breast cancer MDA-MB-231 cells in comparison with the existing antibody and the base chimeric antibody 002_6_c.

[Figure 10] Figure 10 is a diagram showing the AMP degradation suppressive function of 002_6_h4 against a recombinant CD73 antigen.

[Figure 11] Figure 11 is diagrams showing the influence of 002_6_h4 on the division (proliferation) of a human peripheral blood T cells.

[Figure 12] Figure 12 is diagrams showing the AMP degradation suppressive function (H322 cell) of the 003_m antibody to the 009_m antibody prepared in Example 1.

[Figure 13] Figure 13 is a diagram showing the AMP degradation suppressive function (MDA-MB-231 cell) of the four antibodies (004_m, 006_m, 007_m, and 008_m) determined to have high functionality in Figure 12.

[Figure 14-1] Figure 14-1 is a diagram showing amino acid sequences of the heavy chain variable regions of the obtained antibodies (003_m antibody to 009_m antibody).

[Figure 14-2] Figure 14-2 is a diagram showing amino acid sequences of the light chain variable regions of the obtained antibodies (003_m antibody to 009_m antibody).

[Figure 15] Figure 15 is diagrams showing the binding activity of the 003_m antibody to the 009_m antibody to a target expressing cells (H322 cells).

[Figure 16] Figure 16 is diagrams showing the binding activity of the 003_m antibody to the 009_m antibody to a target expressing cells (MDA-MB-231 cells).

[Figure 17] Figure 17 is diagrams showing the binding activity of the 003_m antibody to the 009_m antibody to a target expressing cells (forcibly CD73 expressing HEK-293T cells).

[Figure 18] Figure 18 is a diagram showing results of comparing the dividing cell ratio when the antibody concentration was 1 nM.

[Figure 19] Figure 19 is diagrams showing the binding activity of antibodies to cells expressing the human CD73 gene.

[Figure 20-1] Figure 20-1 is diagrams showing the binding activity of antibodies to cells expressing the human CD73 gene.

[Figure 20-2] Figure 20-2 is diagrams showing the binding activity of antibodies to cells expressing the human CD73 gene.

[Figure 21] Figure 21 is diagrams showing the binding activity of antibodies to cells expressing cynomolgus CD73.

[Figure 22] Figure 22 is diagrams showing that the antibody of the present invention has an inhibitory activity against the AMP degradation action by CD73 on the cell surface.

[Figure 23] Figure 23 is diagrams showing that the antibody of the present invention has a proliferative effect on CD4T cells and CD8T cells, and has the action of increasing the ability to produce IFNγ, TNF, and IL-2 from these cells.

[Figure 24] Figure 24 is a diagram showing fluorescence microscope images indicating the intracellular uptake of the 002_6_h4 antibody and the 006_hKB antibody.

[Figure 25] Figure 25 is diagrams showing that the antibody of the present invention bound to CD73 on the surface of T cells decreases with time (is internalized into the T cells) when these cells are cultured.

[Figure 26] Figure 26 is diagrams showing that the antibody of the present invention bound to CD73 on the surface of cancer cells decreases with time (is internalized into the cancer cells) when these cells are cultured.

[Figure 27] Figure 27 is diagrams showing results of measuring the number of tumor-infiltrating T cells in the tumor on day 35 after administration of the 002_6_h4 antibody or an isotype control antibody.

[Figure 28-1] Figure 28-1 is diagrams showing whether or not the developed antibodies of the present invention enhance the cytolytic activity of T cells against cancer cells.

[Figure 28-2] Figure 28-2 is diagrams showing whether or not the developed antibodies of the present invention enhance the cytolytic activity of T cells against cancer cells.

[Figure 29] Figure 29 is diagrams showing results of an animal experiment investigating whether or not 002_6_h4 can enhance the antitumor effect of the human immune system reproduced in a simulated manner in mice.

[Figure 30] Figure 30 is a diagram showing results of changes in the volume of a tumor mass in immunodeficient mice.

[Figure 31] Figure 31 is a diagram confirming the antitumor effect of the anti-CD73 antibody of the present invention in the body of knock-in mice expressing human CD73.

Description of Embodiments

The present invention can, in one embodiment, provide a binding substance to a CD73 antigen, particularly an antibody or a human-type antibody derivative, that has the binding activity to the CD73 antigen on a cancer cell or immune cells and activates T cells having the toxic activity to cancer cells. The binding substance, particularly an antibody or a human-type antibody derivative, of the present invention can be used in order to treat cancer cells expressing the CD73 antigen, to inhibit the proliferation of cancer cells, and to shrink or eliminate tumor, and can also be used in order to activate T cells having the toxic activity to cancer cells when the T cells are exhausted.

Antibody and human-type antibody derivative

The present invention, in one embodiment, provides an antibody or a human-type antibody derivative that has the binding activity to the CD73 antigen. This antibody or human-type antibody derivative is characterized by activating T cells having the toxic activity to cancer cells.

The antibody or human-type antibody derivative of the present invention may be based on an embodiment that suppresses the function of CD73 expressed in cancer cells and releases the exhaustion of immune cells (particularly T cells) caused by accumulation of an AMP metabolite (adenosine) around the cancer cells and thereby activates the immune cells and exerts tumor proliferation suppressive effect and a cancer treatment effect, be based on an embodiment that suppresses the function of CD73 expressed in immune cells (particularly T cells), activates the immune cells, and exerts tumor proliferation suppressive effect and a cancer treatment effect, or be a cancer treatment effect by controlling the peritumoral environment other than immune cells, or a cancer treatment effect by directly controlling tumor.

In the present invention, when simply referring to an "antibody," the antibody may be derived from any animal species of a mammal, and the species from which the antibody is derived is not limited to human, and may be mouse, rat, guinea pig, hamster, rabbit, or the like.

In the present invention, the antibody may also be a human-type antibody derivative of the antibody described above as long as it has the binding activity to the CD73 antigen and has the functional characteristic of suppressing the cancer. In the present invention, when referring to a human-type antibody derivative, as one embodiment, it is possible to provide an antibody derivative characterized in that the antibody derivative has amino acid sequences of 6 CDRs of the above antibody (heavy chain complementarity determining region (CDR) 1 to CDR3 and light chain CDR1 to CDR3) and an amino acid sequence of the constant region derived from a human antibody and the other amino acid sequences are a combination of an amino acid sequence derived from the original antibody and an amino acid sequence derived from the human antibody. Examples of such an antibody derivative include a humanized antibody in which the region other than the complementarity determination regions (CDRs) of the above "antibody" is replaced with an amino acid sequence derived from a human antibody, or a chimeric antibody in which a variable region of the above antibody is connected to a constant region of a human antibody, a polyvalent antibody in which one antibody has a plurality of antigen binding sites, and a multispecific antibody (bispecific antibody) in which one antibody has a plurality of specificities, and also include other than these antibodies.

The antibody or human-type antibody derivative of the present invention is characterized by having a total of 6 amino acid sequences of the heavy chain CDR1 to 3 and the light chain CDR1 to 3 which are the same as those of the antibody having the binding activity to the CD73 antigen. Examples of the amino acid sequences of 6 CDRs of an antibody having the binding activity to the CD73 antigen that can be considered include one comprising complementarity determining regions of any combination of heavy and light chains selected from the group consisting of the following (1) to (8):

(1) complementarity determining regions of a heavy chain, CDR1 ($DX_1NMD$ (wherein $X_1$ is C or S), SEQ ID No.: 1), CDR2 (DINPNNGGTIYNQKFKG, SEQ ID No.: 2), and CDR3 (TNWDYAMDY, SEQ ID No.: 3) and complementarity determining regions of a light chain, CDR1 ($KASQDINSX_2LS$ (wherein $X_2$ is N, D, or Q), SEQ ID No.: 4), CDR2 (RANRLID, SEQ ID No.: 5), and CDR3 ($X_3QYDVFPRT$ (wherein $X_3$ is L or Q), SEQ ID No.: 6);

(2) complementarity determining regions of a heavy chain, CDR1 (SFGMH, SEQ ID No.: 9), CDR2 (YISSGSRTIYYADTVRG, SEQ ID No.: 10), and CDR3 (DFGSSSPNYFDY, SEQ ID No.: 11), and complementarity determining regions of a light chain, CDR1 (RASESVDNYGISFMN, SEQ ID No.: 12), CDR2 (AASNQGS, SEQ ID No.: 13), and CDR3 (QQSKEVPWT, SEQ ID No.: 14);

(3) complementarity determining regions of a heavy chain, CDR1 (GYWMN, SEQ ID No.: 17), CDR2 (RIDPYDSETHYSQKFKD, SEQ ID No.: 18), and CDR3 (SSPITTAPFDY, SEQ ID No.: 19), and complementarity determining regions of a light chain, CDR1 (RASESVDYYGFSFMN, SEQ ID No.: 20), CDR2 (AASTQGS, SEQ ID No.: 21), and CDR3 (QQSKEVPYT, SEQ ID No.: 22);

(4) complementarity determining regions of a heavy chain, CDR1 (SYGVS, SEQ ID No.: 25), CDR2 (VIWGDGSTNYHSALIS, SEQ ID No.: 26), and CDR3 (TNIFYDYDWYLDV, SEQ ID No.: 27), and

complementarity determining regions of a light chain, CDR1 (RSSQSLVHSNGNTYLH, SEQ ID No.: 28), CDR2 (KVSNRFS, SEQ ID No.: 29), and CDR3 (SHSTHVPWT, SEQ ID No.: 30);

(5) complementarity determining regions of a heavy chain, CDR1 (SYWMH, SEQ ID No.: 33), CDR2 (EINPSNARTNYNENFKS, SEQ ID No.: 34), and CDR3 (RGTSGNYFDF, SEQ ID No.: 35), and

complementarity determining regions of a light chain, CDR1 (KASQDINTYLS, SEQ ID No.: 36), CDR2 (RANRLVD, SEQ ID No.: 37), and CDR3 (LQYDEFPYT, SEQ ID No.: 38);

(6) complementarity determining regions of a heavy chain, CDR1 (SYWMN, SEQ ID No.: 41), CDR2 (KIDPYDSETHYNQKFKD, SEQ ID No.: 42), and CDR3 (IRYGTFDY, SEQ ID No.: 43), and

complementarity determining regions of a light chain, CDR1 (KASQDINNYLS, SEQ ID No.: 44), CDR2 (RANILVD, SEQ ID No.: 45), and CDR3 (LQYDEFPYT, SEQ ID No.: 46);

(7) complementarity determining regions of a heavy chain, CDR1 (SYWMH, SEQ ID No.: 49), CDR2 (EINPSNGRTNYNEKFKN, SEQ ID No.: 50), and CDR3 (RGTSGNYFDY, SEQ ID No.: 51), and

complementarity determining regions of a light chain, CDR1 (KASQDINTYLS, SEQ ID No.: 52), CDR2 (RANRLVD, SEQ ID No.: 53), and CDR3 (LQYDEFPYT, SEQ ID No.: 54); and

(8) complementarity determining regions of a heavy chain, CDR1 (SYWMN, SEQ ID No.: 57), CDR2 (RIDPYDSEAHYNQKFKD, SEQ ID No.: 58), and CDR3 (IRYGTFDY, SEQ ID No.: 59), and

complementarity determining regions of a light chain, CDR1 (KASQDINSYLS, SEQ ID No.: 60), CDR2 (RSNSLVD, SEQ ID No.: 61), and CDR3 (LQYDEFPYT, SEQ ID No.: 62);

and an antibody or human-type antibody derivative specified by a CDR combination other than the above CDR combination is also included as long as it has the characteristic of having the binding activity to the CD73 antigen.

The antibody or human-type antibody derivative of the present invention is also characterized by having the ability to activate T cells. In particular, it is preferable to suppress the function of CD73 expressed in cancer cells or immune cells and release the exhaustion of the immune cells (particularly T cells) and thereby activate the immune cells and exert a cancer proliferation suppressive effect and a cancer treatment effect. Such activation of T cells may occur in vitro or in vivo. Whether or not an antibody has the ability to activate such T cells having the toxic activity to cancer cells can be determined by screening antibodies having the binding activity to the CD73 antigen to see if they actually activate T cells.

In the present invention, the exhaustion of immune cells (particularly T cells) refers to a state in which the immune cells has become dysfunctional. Specific examples thereof include a decrease in cytokine secretion ability and cytotoxicity in T cells, a decrease in cytokine secretion ability and cytotoxicity in NK cells, a decrease in antigen presentation ability of dendritic cells, and activation of regulatory T cells. In this state, the cancer immunity of an individual to cancer cells does not function or functions poorly, and the activity of eliminating cancer cell decreases. It is known that such exhaustion of immune cells is caused by the increase in a checkpoint protein such as PD-1 or CTLA-4 on the surface of T cells, and is generated in effector T cells when cancer cells that continue to proliferate forces the immune system to be active for a long period of time, but the exhaustion is a reversible state, and it is considered that the state of exhaustion can also be released by activating the same cells.

In the present invention, when referring to activation of T cells, the activation can be determined by using proliferation of T cells, an enhancement of cytotoxicity of T cells against cancer cells, cytokine secretion from T cells, or the like as an indicator. When the activation of T cells is observed, it indicates that the antibody or human-type antibody derivative of the present invention inhibits the activity of CD73 in the T cells and suppresses the reaction that degrades AMP into adenosine.

Screening for the ability of the antibody or human-type antibody derivative of the present invention to activate T cells can be carried out in vivo or in vitro. In vivo screening can be carried out by transplanting target cancer cells into animals such as wild-type mice or mice in which immune cells are transplanted into immunodeficient mice such as nude mice or SCID mice and measuring a change in the size of tumor mass in the body when the antibody or human-type antibody derivative of the present invention is administered, or by anatomically investigating the infiltration of T cells around tumor mass in the body when the antibody or human-type antibody derivative of the present invention is administered. In vitro screening can be carried out by contacting peripheral blood T cells with the antibody or human-type antibody derivative of the invention under a culture condition to measure proliferation of the T cells, measure cytokine secretion of the T cells, or investigate whether or not the T cells causes cell death of cancer cells.

In one embodiment, the antibody or human-type antibody derivative of the present invention can be specified as one having the amino acid sequences of the following heavy chain variable region VH domain: (1) 002_m heavy chain variable region (SEQ ID No.: 7), (2) 003_m heavy chain variable region (SEQ ID No.: 15), (3) 004_m heavy chain variable region (SEQ ID No.: 23), (4) 005_m heavy chain variable region (SEQ ID No.: 31), (5) 006_m heavy chain variable region (SEQ ID No.: 39), (6) 007_m heavy chain variable region (SEQ ID No.: 47), (7) 008_m heavy chain variable region (SEQ ID No.: 55), and (8) 009_m heavy chain variable region (SEQ ID No.: 63). Here, the numbers (1) to (8) correspond to the numbers of the combinations of the sequences of the heavy chain CDR1 to CDR3 described above. For example, the amino acid sequence of SEQ ID NO: 7 is a sequence comprising the amino acid sequences of the heavy chains CDR1 to CDR3 specified by SEQ ID NO: 1 to SEQ ID NO: 3.

In one embodiment, the antibody or human-type antibody derivative of the present invention can be specified as one having the amino acid sequences of the following light chain variable region VL domain: (1) 002_m light chain variable region (SEQ ID No.: 8), (2) 003_m light chain variable region (SEQ ID No.: 16), (3) 004_m light chain variable region (SEQ ID No.: 24), (4) 005_m light chain variable region (SEQ ID No.: 32), (5) 006_m light chain variable region (SEQ ID No.: 40), (6) 007_m light chain variable region (SEQ ID No.: 48), (7) 008_m light chain variable region (SEQ ID No.: 56), and (8) 009_m light chain variable region (SEQ ID No.: 64). Here, the numbers (1) to (8) correspond to the numbers of the combinations of the sequences of the light chain CDR1 to CDR3 described above. For example, the amino acid sequence of SEQ ID NO: 8 is a sequence comprising the amino acid sequences of the light chains CDR1 to CDR3 specified by SEQ ID NO: 4 to SEQ ID NO: 6.

In the present invention, the human-type antibody derivative of the present invention also includes a functional fragment of the antibody or human-type antibody derivative described above. Examples of the functional fragment of the antibody or human-type antibody derivative in the present invention include F(ab')2, Fab', Fab, and single chain Fv (scFv). The functional fragment of the present invention may be any fragment of the above functional fragments as long as it is characterized by being able to induce the toxic activity to cancer cells, and for example, an F(ab')2 fragment or the like can be used as such a functional fragment.

Obtaining antibody or human-type antibody derivative

The antibody or human-type antibody derivative of the present invention can also be obtained by administering the CD73 antigen as an immunogen to animals of the above species, collecting an antibody-producing cells from the animals and culturing the antibody-producing cells, and may be recombinantly obtained by designing a vector for protein expression including a DNA sequence that can define the amino acid sequence of the antibody or human-type antibody derivative and introducing the vector into cells for protein production.

The DNA sequence that can define the amino acid sequence of the antibody or human-type antibody derivative of the present invention can be prepared by a method involving obtaining the DNA sequence from cells producing the target antibody or human-type antibody derivative, a method involving designing the DNA sequence based on a codon optimized for the animal species used in an expression system based on the amino acid sequence, or a method using a combination of these methods.

The antibody or human-type antibody derivative of the present invention can be obtained by using a method well known to those skilled in the art, such as incorporating the prepared DNA sequence into an expression vector suitable for cell types for protein expression (for example, a CHO cell) in which the antibody or human-type antibody derivative is to be expressed and introducing the vector into the cell types for protein expression.

In the case of the antibody, the antibody has a structure in which a heavy chain and a light chain are combined, and thus can be prepared, for example, by a method involving introducing a vector comprising a DNA sequence that defines a heavy chain amino acid sequence and a vector comprising a DNA sequence that defines a light chain amino acid sequence into cell types for protein expression and expressing both proteins in the cells to produce the antibody in the cells, or a method involving introducing a vector comprising both a DNA sequence that defines a heavy chain amino acid sequence and a DNA sequence that defines a light chain amino acid sequence into cell types for protein expression and expressing both proteins in the cell to produce the antibody in the cells.

Examples of the human-type antibody derivative obtained as one embodiment in the present invention include, but are not limited to,

an antibody comprising the 002_6_h1 heavy chain variable region (SEQ ID NO: 65) and the 002_6_h1 light chain variable region (SEQ ID NO: 66),
an antibody comprising the 002_6_h2 heavy chain variable region (SEQ ID NO: 67) and the 002_6_h2 light chain variable region (SEQ ID NO: 68),
an antibody comprising the 002_6_h3 heavy chain variable region (SEQ ID NO: 69) and the 002_6_h3 light chain variable region (SEQ ID NO: 70),
an antibody comprising the 002_6_h4 heavy chain variable region (SEQ ID NO: 71) and the 002_6_h4 light chain variable region (SEQ ID NO: 72),
an antibody comprising the 002_6_h5 heavy chain variable region (SEQ ID NO: 73) and the 002_6_h5 light chain variable region (SEQ ID NO: 74),
an antibody comprising the 002_6_h6 heavy chain variable region (SEQ ID NO: 75) and the 002_6_h6 light chain variable region (SEQ ID NO: 76),
an antibody comprising the 002_6_h7 heavy chain variable region (SEQ ID NO: 77) and the 002_6_h7 light chain variable region (SEQ ID NO: 78),

an antibody comprising the 002_6_h8 heavy chain variable region (SEQ ID NO: 79) and the 002_6_h8 light chain variable region (SEQ ID NO: 80),

an antibody comprising the 004_h3_ heavy chain variable region (SEQ ID NO: 81) and the 004_h3_ light chain variable region (SEQ ID NO: 82),

an antibody comprising 006_hKB_ heavy chain variable region (SEQ ID NO: 83) and the 006_hKB_ light chain variable region (SEQ ID NO: 84),

an antibody comprising the 007_h1_ heavy chain variable region (SEQ ID NO: 85) and the 007_h1_ light chain variable region (SEQ ID NO: 86), and

an antibody comprising the 008_h4_ heavy chain variable region (SEQ ID NO: 87) and the 008_h4_ light chain variable region (SEQ ID NO: 88),

when the human-type antibody derivative is prepared from the above antibody (1),

and a human-type antibody derivative prepared from another antibody is also included as an embodiment of the present invention.

Use of antibody or human-type antibody derivative

Based on the above characteristic of having the ability to activate immune cells (particularly T cells) having cytotoxicity against cancer cells, the antibody or human-type antibody derivative of the present invention can, in one embodiment, provide a pharmaceutical composition comprising the antibody or human-type antibody derivative of the invention that activates an immune cell (particularly T cells) having the toxic activity to cancer cells in a subject in need of treatment or prevention of a cancer.

Examples of the cancer cells that can be targeted in the present invention include cells derived from a cancer selected from the group consisting of leukemia (including chronic lymphocytic leukemia and acute lymphocytic leukemia), lymphoma (including non-Hodgkin's lymphoma, Hodgkin's lymphoma, T cell line lymphoma, B cell line lymphoma, Burkitt's lymphoma, malignant lymphoma, diffuse lymphoma, and follicular lymphoma), myeloma (including multiple myeloma), melanoma, lung cancer, breast cancer, colorectal cancer, kidney cancer, stomach cancer, ovarian cancer, pancreatic cancer, cervical cancer, uterine body cancer, endometrial cancer, esophageal cancer, liver cancer, head and neck cancer, head and neck squamous cell cancer, skin cancer, urinary tract cancer, prostate cancer, chorionic cancer, pharyngeal cancer, laryngeal cancer, thecoma, arrhenoblastoma, endometrial hyperplasia, endometriosis, embryoma, fibrosarcoma, Kaposi's sarcoma, hemangioma, cavernous hemangioma, hemangioblastoma, retinoblastoma, astrocytoma, neurofibroma, oligodendroglioma, medulloblastoma, neuroblastoma, glioma, rhabdomyosarcoma, glioblastoma, osteogenic sarcoma, leiomyosarcoma, thyroid sarcoma, and Wilms tumor. In the embodiment of the present invention, the cancer cells are preferably cancer cells expressing the CD73 antigen, and are preferably cells derived from, for example, melanoma, breast cancer, lung cancer, or colorectal cancer.

The antibody or human-type antibody derivative of the present invention is characterized by activating immune cells (particularly T cells) having cytotoxicity against target cancer cells as described above. That is, the antibody or human-type antibody derivative of the present invention is required to cause the activation of immune cells (particularly T cells) only in immune cells having cytotoxicity against cancer cells expressing the target CD73 antigen when administered to a living body, and not to induce cytotoxicity that may cause a clinical problem when cells express the CD73 antigen but is a normal cell.

In the present invention, the antibody or human-type antibody derivative of the present invention can also be provided as a composition in combination with other antibodies or other agents such as an anticancer agent. In one embodiment, the antibody or human-type antibody derivative of the present invention can also be bound to a drug to form an antibody drug conjugate (ADC).

In the present invention, it is also possible to provide a formulation comprising the antibody or human-type antibody derivative of the present invention together with a physiologically acceptable diluent or carrier. Examples of a suitable carrier include, but are not limited to, one including a buffer (phosphate buffer solution, citrate buffer solution, acetate buffer solution, or the like), a salt (sodium chloride, or the like), a sugar (glucose, trehalose, mannitol, sorbitol, or the like), and an additive (an amino acid such as arginine, a surfactant such as a polysorbate, or the like). Alternatively, the antibody or human-type antibody derivative of the present invention can also be freeze-dried, and reconstituted by adding a buffered aqueous solution as described above when necessary, before use. The formulation comprising the antibody or human-type antibody derivative of the present invention can be administered in various dosage forms, and can be, for example, a parenteral administration agent such as an injection or an infusion.

The dosage of the antibody or human-type antibody derivative of the present invention varies depending on the symptom, the age, the body weight, or the like, but usually, in parenteral administration, the antibody or human-type antibody derivative can be administered via an appropriate route of administration such as intraperitoneal injection, subcutaneous injection, intramuscular injection, intratumor injection, or intravenous injection depending on the type of the cancer in the range of 0.01 mg to 1000 mg per kg of body weight per time, preferably 0.05 mg to 500 mg, 0.05 mg to 100 mg, 0.05 mg to 50 mg, 0.05 mg to 20 mg, or the like per kg of body weight per day, and more preferably in the range of 0.1 mg to 10 mg per kg of body weight per day.

Based on the above characteristic of having the ability to activate immune cells (particularly T cells) having cytotoxicity against cancer cells, the present invention can, in another embodiment, also provide a method for treating or preventing a cancer in a subject in need of treatment or prevention of the cancer, comprising administering an effective amount of the antibody or human-type antibody derivative of the present invention to the subject. The treatment or prevention of cancer with the antibody or human-type antibody derivative of the present invention is caused by activating immune cells (particularly T cells) having cytotoxicity against cancer cells in vivo by this antibody or human-type antibody derivative.

When the antibody or human-type antibody derivative of the present invention is administered to a living body for the treatment or prevention of cancer, the antibody or human-type antibody derivative can be administered together with an adjuvant (for example, one disclosed in Clin. Microbiol. Rev., 7:277-289, 1994) so that cell-mediated immunity or humoral immunity is effectively established in vivo, and can also be administered in a particulate dosage form such as a liposome formulation, a particulate formulation obtained by binding the antibody or human-type antibody derivative to a bead having a diameter of several micrometers, or a formulation obtained by binding a lipid to the antibody or human-type antibody derivative.

In vitro use of antibody and human-type antibody derivative

The antibody or human-type antibody derivative of the present invention can be used in order to detect the CD73 antigen in a sample based on the characteristic of having the binding activity to the CD73 antigen. Specifically, the antibody or human-type antibody derivative of the present invention can be used when carrying out various methods for detecting the CD73 antigen that can be carried out using an antibody such as a purification method using an antibody such as an immunoprecipitation method, an agglutination reaction, or a magnetic bead method, an ELISA method, Western blot, an immunoassay such as immunohistochemistry, or immunocytochemistry such as flow cytometry on a sample including cancer cells or immune cells (for example, T cells) collected from a subject. In each case, the antibody or human-type antibody derivative of the present invention can be detected using a label for detection (for example, fluorescence or an enzyme) generally known to those skilled in the art.

Based on the characteristic of having the binding activity to the CD73 antigen and activating immune cells (particularly T cells) having the toxic activity to cancer cells, the antibody or human-type antibody derivative of the present invention can also be used in order to measure the cytotoxicity of the antibody or human-type antibody derivative of the present invention against cancer cells in a subject. Examples of a method for measuring such cytotoxicity against cancer cells in a subject include a method for measuring cytotoxicity against cancer cells using the antibody or human-type antibody derivative of the present invention comprising the following steps:

a step of contacting cancer cells collected from a subject with the antibody or human-type antibody derivative of the present invention under a culture condition (that is, in vitro), and
a step of measuring whether or not AMP metabolism (reaction that degrades AMP into adenosine) of the cancer cells is reduced, a step of measuring whether or not cell viability is reduced, or a step of measuring whether or not secretion of an immunostimulatory substance is increased, under a culture condition.

A phenomenon in which when AMP metabolism (reaction that degrades AMP into adenosine) in cancer cells is reduced in vivo, T cells are activated and a tumor is shrunk is known, and it is thought that cancer immunity to the cancer cell is activated. Utilizing this phenomenon, in one embodiment of the present invention, cytotoxicity against cancer cells can be measured by contacting cancer cells collected from a subject with the antibody or human-type antibody derivative of the present invention in vitro, and measuring whether or not the AMP metabolism of the cancer cells is reduced under a culture condition. More specifically, by contacting cancer cells collected from a subject with the antibody or human-type antibody derivative of the present invention and measuring the reaction of degradation from AMP into adenosine under a culture condition (that is, in vitro), the cytotoxicity against the cancer cells in the subject (in vivo) when the antibody or human-type antibody derivative of the present invention is administered to the subject can be measured.

In this embodiment, the reaction of degradation from AMP into adenosine can be measured by an HPLC method, an LC-MS method, an ELISA method, or an AMP-dependent chemiluminescence method (Cell titer glo or the like).

In vivo, a peripheral blood lymphocyte causes a decrease in the cell viability of cancer cells. Utilizing this phenomenon, in one embodiment of the present invention, by contacting cancer cells collected from a subject with the antibody or human-type antibody derivative of the present invention in vitro, and measuring whether or not the cell viability is reduced under a culture condition, the cytotoxicity against the cancer cells can be measured. More specifically, by contacting cancer cells collected from a subject with the antibody or human-type antibody derivative of the present invention in the presence of peripheral blood lymphocytes of the same subject and measuring whether or not the cell viability of the cancer cells is reduced under a culture condition (that is, in vitro), the cytotoxicity against the cancer cells in the subject (in vivo) when the antibody or human-type antibody derivative of the present invention is administered to the subject can be measured.

A phenomenon in which when the secretion of an immunostimulatory substance from peripheral blood lymphocytes is increased in vivo, T cells are activated and a tumor is shrunk is known, and it is thought that cancer immunity to cancer cells is activated. Utilizing this phenomenon, in one embodiment of the present invention, the cytotoxicity against cancer cells can be measured by contacting the cancer cells collected from a subject with the antibody or human-type antibody derivative of the present invention in vitro, and measuring whether or not the secretion of an immunostimulatory substance is increased under a culture condition. More specifically, by contacting cancer cells collected from a subject with the antibody or human-type antibody derivative of the present invention in the presence of peripheral blood lymphocytes of the same subject and measuring whether or not the secretion of an immunostimulatory substance from the peripheral blood lymphocytes is increased under a culture condition (that is, in vitro), the activation of immune cells such as lymphocytes having cytotoxicity in the subject (in vivo) and the resulting increase in cytotoxicity against the cancer cells when the antibody or human-type antibody derivative of the present invention is administered to the subject can be measured.

In this embodiment, examples of the immunostimulatory substance to be measured for determining cytotoxicity against cancer cells include IFN-$\gamma$ and TNF$\alpha$. IFN$\gamma$ is known to activate immune cells such as an NK cells and cause the cells to exert an antitumor effect.

The present invention can also provide a measurement kit comprising the antibody or human-type antibody derivative of the present invention for in vitro measuring AMP metabolism, cytotoxicity, secretion of an immunostimulatory substance, or activation of immune cells against cancer cells collected from a subject.

The measurement kit for measuring AMP metabolism can comprise, in addition to the antibody or human-type antibody derivative of the present invention, AMP as a substrate, a reagent for measuring adenosine, which is a metabolite thereof (AMP-dependent chemiluminescent reagent, ELISA reagent) and an apparatus (HPLC system, LC-MS system).

The measurement kit for measuring cytotoxicity can comprise, in addition to the antibody or human-type antibody derivative of the present invention, known means for measuring cell proliferation properties (for example, measurement of uptake of thymidine, uptake of BrdU, free lactate dehydrogenase (LDH) activity, or measurement of a living cell-derived substance (reductase activity, esterase activity, or ATP).

The measurement kit for measuring the secretion of an immunostimulatory substance can comprise, in addition to the antibody or human-type antibody derivative of the present invention, means for detecting an immunostimulatory substance to be measured (for example, a primary antibody against the immunostimulatory substance and a secondary antibody for the detection of the primary antibody).

The measurement kit for measuring activation of immune cells can comprise, in addition to the antibody or human-type antibody derivative of the present invention, a labeling reagent for measuring the division of immune cells using a flow cytometer.

Hereinafter, the present invention will be specifically described with reference to Examples. The Examples shown below do not limit the invention in any way.

Examples

Example 1: Preparation of antibody

The present Example was carried out for the purpose of obtaining a monoclonal antibody against the CD73 antigen using mice.

(1-1) Preparation of antibody by B cell single cell sorting method

10 $\mu$g of a CD73 recombinant antigen (self-prepared product) was administered intraperitoneally to mice (BALB/cAJcl female 7 weeks old, CLEA Japan, Inc.) 4 to 8 times every 2 weeks for immunization. One week after the final administration, blood was collected from the tail vein, and the increase in antibody titer specific to the CD73 antigen in the blood was confirmed by antigen-immobilized ELISA based on the conventional method, and then the spleen of a mouse having an antibody having a significantly elevated antibody titer specific to the CD73 antigen was recovered.

A single cell suspension of spleen cells was prepared from the spleen according to a known method, and B cells were purified using a MACS-pan B cell isolation kit (Miltenyi 130-104-443). The purified B cells were stained with ZOMBIE-APC-Cy7 (Biolegend), Alexa488 anti-IgM (Biolegend), VB421anti-IgG (Biolegend), and PE-CD73 (self-prepared product) and subjected to FACS Aria (BD), and an IgG positive and CD73 antigen-binding cell population was separated into a 96 well plate at one cell per well.

The genes corresponding to the heavy chain variable region and the light chain variable region of the antibody were amplified for each well by the RT-PCR method. As a primer used for the amplification, based on the information in known literature, a sequence comprising the 5' end and 3' end regions of each of the heavy chain variable region and the light chain variable region was adopted, and a mix primer having a plurality of sequences was used in consideration of the diversity of the antibody gene sequence (heavy chain: H1 mix and H2 mix, light chain: k mix) (Lotta von Boehmer et al., Nature Protocols, vol 11, p.1908-1923 (2016)).

After amplification of the heavy chain gene and the light chain gene of the antibody, a linker sequence for insertion into an antibody expression vector was added to an end of each of the genes by a PCR reaction, and inserted into a vector for heavy chain expression or a vector for light chain expression (a CMV promoter, a secretory signal, the gene of the heavy chain variable region or the light chain variable region, respectively, and the gene of the heavy chain constant region or the light chain constant region, respectively, tandemly linked) by a ligation reaction.

The ligation product was transformed into Escherichia coli and the plasmid for antibody expression was recovered, and then a CHO cells were transfected with a heavy chain gene expression plasmid (derived from the H1 or H2 mix amplification product) and a light chain gene expression plasmid (derived from the k mix amplification product). Antibodies produced in the culture supernatant from the CHO cells were recovered, and the antigen specificity of each of the antibodies was confirmed by antigen-immobilized ELISA.

(1-2) Antibody purification

For antibodies whose antigen (CD73) binding activity was confirmed by antigen-immobilized ELISA, the culture supernatant of the CHO cells was recovered, and the antibodies in the culture supernatant were purified using Monospin ProG Column (GL Sciences Inc.).

(1-3) Antibody function screening

The human lung cancer cell line H322 (ATCC(registered trademark) CRL-5806™) was seeded on a 96 well plate, the purified antibodies were added, and the resulting mixture was incubated at 37°C for 1 hour. AMP (Sigma, A1752), which is a substrate of CD73, was added such that a final concentration of 200 $\mu$M was reached, and the resulting mixture was incubated overnight at 37°C to carry out the reaction of degradation of AMP into adenosine by CD73.

The supernatant was recovered, ATP (Sigma, A2383, final concentration of 100 $\mu$M) and Cell TiterGlo (Promega, G9243) were added and mixed, and then the chemiluminescence was measured using a microplate reader. That is, using the principle that AMP inhibits the chemiluminescence due to the reaction between ATP and Cell TiterGlo, the enzyme activity inhibitory effect of the antibodies was evaluated from the amount of residual AMP.

As a positive control, MEDI9447, which is a previously developed product (an antibody having the same sequences as the published variable regions of MEDI9447 was produced using a genetic recombination technology based on the sequence published in Japanese Translation of PCT International Application Publication No. 2018-501197; hereinafter referred to as the "benchmark antibody"), was adopted.

As a result of antibody function screening, eight mouse antibodies named 002_m, 003_m, 004_m, 005_m, 006_m, 007_m, 008_m, and 009_m oc1ld be selected as CD73-binding activity positive antibodies.

Example 2: Antibody function evaluation (concentration-dependent evaluation)

The present Example was carried out for the purpose of evaluating the function of the 002_m antibody obtained in Example 1.

The antibody function evaluation was carried out by the same method as in the antibody function screening described in Example 1 (1-3). That is, human lung cancer cell line H322 cells or human breast cancer cell line MDA-MB-231 cells (ATCC(registered trademark) HTB-26™) were used as cells. In the present Example, the antibody concentration dependence of the AMP degradation suppressive function was analyzed by measuring the AMP degradation reaction in the same manner as in Example 1 (1-3), the IC50 value (50% inhibition concentration: here, refers to the antibody concentration that can inhibit 500 of the enzyme function of CD73) was calculated, and the function of the antibody was quantitatively evaluated.

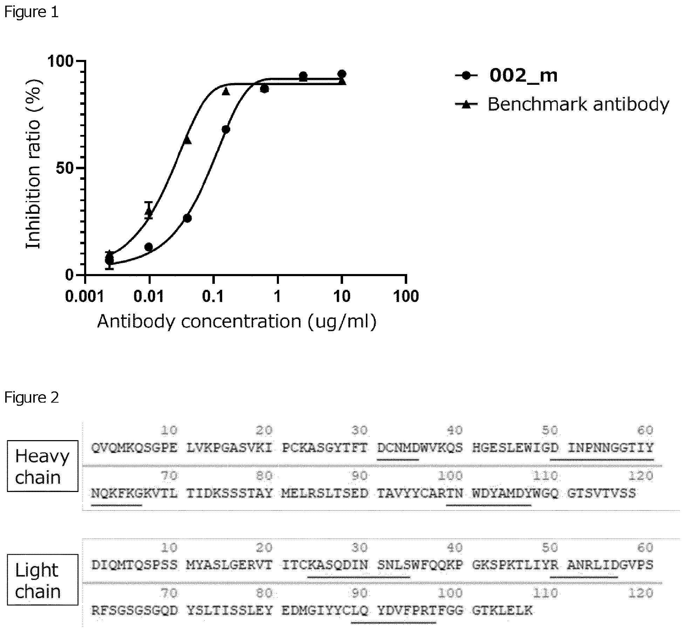

The AMP degradation suppressive function (H322 cells) of the 002_m antibody prepared in Example 1 is shown in Figure 1. This antibody, which is a mouse antibody, was found to show an IC50 value close to that of the benchmark antibody (see Table 1).

[Table 1]

| Sample | IC50 value ($\mu$g/ml) |
|---|---|
| 002_m antibody | 0.089 |
| Benchmark antibody | 0.020 |

Example 3: Antibody gene sequence analysis

The present Example was carried out for the purpose of determining the amino acid sequence of the 002_m antibody obtained in Example 1.

For a 002_m antibody clone whose AMP degradation suppressive function was confirmed by functional analysis, a single colony was recovered from an inoculated plate of Escherichia coli transfected with the ligation product prepared in Example 1 (1-1), and plasmid DNA was obtained.

The obtained plasmid DNA was analyzed for the gene sequences defining the heavy chain variable region and the light chain variable region by Sanger sequencing, and the amino acid sequences were determined based on these gene sequences.

The amino acid sequences of the heavy chain variable region and the light chain variable region of the 002_m antibody are shown in Figure 2 (SEQ ID NO: 7 and SEQ ID NO: 8, respectively). In addition, in this figure, the heavy chain CDR1, CDR2, and CDR3 (SEQ ID NO: 1, SEQ ID NO: 2, and SEQ ID NO: 3, respectively) and the light chain CDR1, CDR2, and CDR3 (SEQ ID NO: 4, SEQ ID NO: 5, and SEQ ID NO: 6, respectively) are underlined.

Example 4: Preparation of human-mouse human chimeric antibody

The present Example was carried out for the purpose of preparing a chimeric antibody having a variable region of a mouse antibody and a constant region of a human antibody for the 002_m antibody sequenced in Example 3.

The gene sequences of the variable regions of the mouse antibody were artificially synthesized and incorporated into a vector for human constant region (Fc) chimeric recombinant antibody expression. As the vector for expression, a plasmid having a light chain expression element (an EF-1$\alpha$ promoter, a secretory signal, a DNA sequence defining the light chain variable region, and a DNA sequence defining the light chain constant region tandemly linked) and a heavy chain expression element (an EF-1$\alpha$ promoter, a secretory signal, a DNA sequence defining the heavy chain variable region, and a DNA sequence defining the heavy chain constant region tandemly linked) was constructed. ExpiCHO cells (Thermo) were transfected with the plasmid and caused to secrete the antibody into the medium, and then the antibody was purified using ProteinA Column (GL Sciences Inc.).

The chimeric antibody thus prepared was defined as the 002_1_c antibody. The amino acid sequence of the heavy chain variable region of this antibody is composed of the amino acid sequence of SEQ ID NO: 7, and the amino acid sequence of the light chain variable region is composed of the amino acid sequence of SEQ ID NO: 8.

Example 5: Preparation of CDR-modified antibody

The present Example was carried out for the purpose of preparing a modified antibody of the 002_m antibody in which a residue in a CDR that could undergo post-translational modification was replaced with a residue that could not undergo modification.

Specifically, the amino acid cysteine (C) at position 32 of the 002_m antibody heavy chain variable region (SEQ ID NO: 7) was replaced with serine (S), and the amino acid asparagine (N) at position 32 of the 002_m antibody light chain variable region (SEQ ID NO: 8) was replaced with aspartic acid (D) or glutamine (Q) (see Table 2 below).

[Table 2]

| Name of antibody | Heavy chain variable region/light chain variable region |
|---|---|
| 002_1_c antibody | SEQ ID NO: 7/SEQ ID NO: 8 |
| 002_2_c antibody | SEQ ID NO: 7/SEQ ID NO: 8(N32D) |
| 002_3_c antibody | SEQ ID NO: 7/SEQ ID NO: 8(N32Q) |
| 002_4_c antibody | SEQ ID NO: 7(C32S)/SEQ ID NO: 8 |
| 002_5_c antibody | SEQ ID NO: 7(C32S)/SEQ ID NO: 8(N32D) |
| 002_6_c antibody | SEQ ID NO: 7(C32S)/SEQ ID NO: 8(N32Q) |

A gene including the relevant regions was artificially synthesized and inserted into a vector for human Fc chimeric recombinant antibody expression by ligation. Antibody expression and purification were carried out in the same manner as for the human chimeric antibody.

Analysis of the binding activity of each of the obtained chimeric antibodies 002_1_c to 002_6_c to target expressing cells was carried out by FACS analysis using a CD73 native expressing cells (human lung cancer cell line H322). The subject antibody or an isotype control antibody was reacted with the cells, then the cells were stained with a fluorescently labeled secondary antibody and subjected to flow cytometry analysis, and the antibody binding activity was evaluated based on the obtained fluorescence intensity.

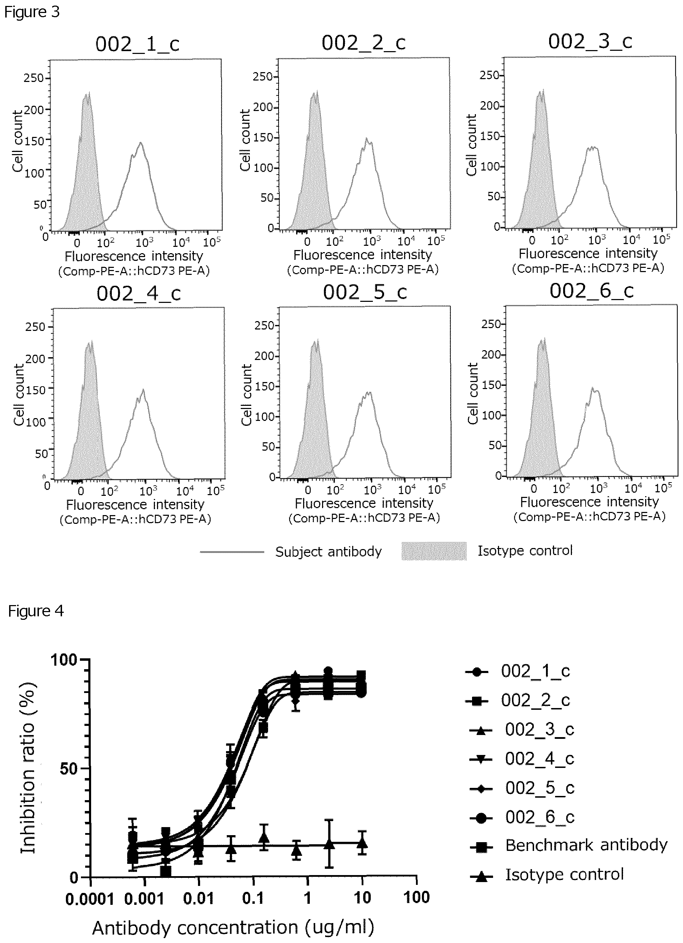

Results are shown in Figure 3. It was found that the chimeric antibodies prepared based on the 002_m antibody in Example 4 and the present Example each show a high binding activity to H322 cells.

Next, for each of the chimeric antibodies, antibody function screening involving measuring the AMP degradation suppressive function of the 002_1_c antibody to the 002_6_c antibody using a human lung cancer H322 cells was carried out by the same method as in (1-3). Results are shown in Figure 4.

From the present results, the IC50 value of each antibody was calculated and found to be as shown in Table 3 below, and it was determined that the binding activity and the functionality of 002_1_c to 002_6_c were all the same. In addition, the following result was also obtained: the IC50 value of each antibody was comparable to the IC50 value of the benchmark antibody. Therefore, it was decided to carry out the subsequent development based on 002_6_c, which does not have a possibility of posttranslation modification.

[Table 3]

| Name of antibody | IC50 value ($\mu$g/ml) |
|---|---|
| 002_1_c | 0.042 |
| 002_2_c | 0.077 |
| 002_3_c | 0.039 |
| 002_4_c | 0.038 |
| 002_5_c | 0.066 |
| 002_6_c | 0.042 |
| Benchmark antibody | 0.040 |

Example 6: Preparation of humanized antibody

The present Example was carried out for the purpose of preparing a humanized antibody based on the 002_6_c antibody (SEQ ID NO: 7 (C32S)/SEQ ID NO: 8 (N32Q)) selected in Example 5.

The humanized antibody was prepared using the public domain software Tabhu (http://circe.med.uniromal.it/tabhu/). Using this software, sequences having a property similar in sequence and conformation to 002_6_c were extracted from a human antibody database, 4 sequences (FJ039788, AF146404, 3SQO_H, and AY686911) were chosen as candidate sequences, and the sequences other than the CDR regions of these antibodies were grafted with the sequences of the CDR regions of 002_6_c.

Next, backmutation of some amino acid residues to the mouse sequence was carried out. The amino acid residues to be backmutated were selected with an upper limit of 10 amino acid residues for each sequence based on the large structural difference between the humanized antibody and the mouse antibody (higher TubHu score). For details, the method described in Non Patent Literature (Bioinformatics. 2015 Feb 1;31(3):434-5) was followed.

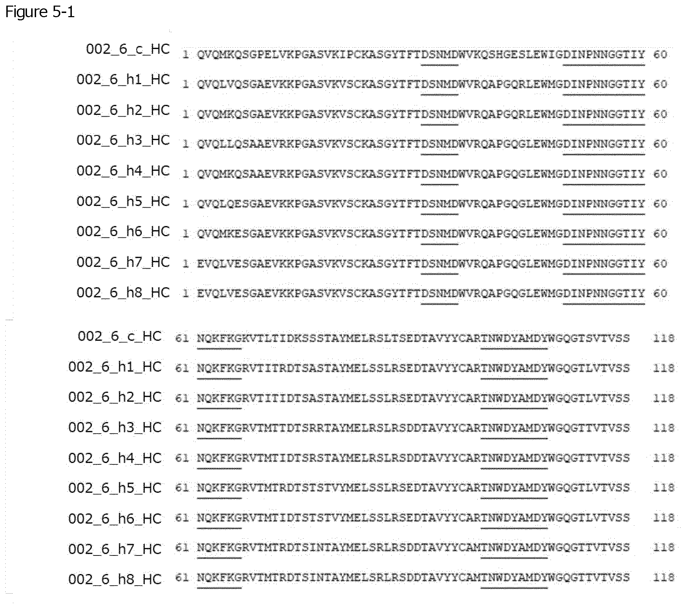

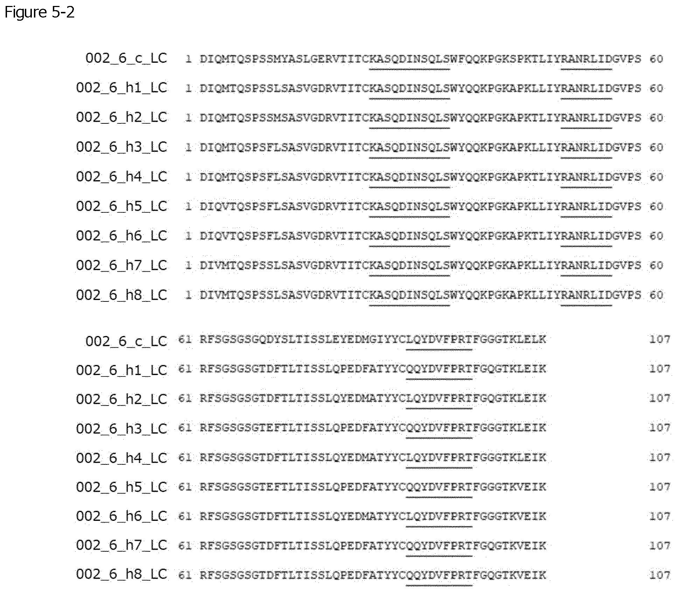

The amino acid sequences of the variable regions of the obtained humanized antibodies (002_6_h1 to 002_6_h8) of 002_6_c are shown in Figure 5 (the heavy chain variable regions are shown in Figure 5-1 and the light chain variable regions are shown in Figure 5-2). Each amino acid sequence was assigned SEQ ID NO: as shown in Table 4 below.

[Table 4]

| Sequence overview | SEQ ID NO: | Sequence overview | SEQ ID NO: |
|---|---|---|---|
| 002_6_h1 heavy chain variable region | 65 | 002_6_h5 heavy chain variable region | 73 |
| 002_6_h1 light chain variable region | 66 | 002_6_h5 light chain variable region | 74 |
| 002_6_h2 heavy chain variable region | 67 | 002_6_h6 heavy chain variable region | 75 |

(continued)

| Sequence overview | SEQ ID NO: | Sequence overview | SEQ ID NO: |
|---|---|---|---|
| 002_6_h2 light chain variable region | 68 | 002_6_h6 light chain variable region | 76 |
| 002_6_h3 heavy chain variable region | 69 | 002_6_h7 heavy chain variable region | 77 |
| 002_6_h3 light chain variable region | 70 | 002_6_h7 light chain variable region | 78 |
| 002_6_h4 heavy chain variable region | 71 | 002_6_h8 heavy chain variable region | 79 |
| 002_6_h4 light chain variable region | 72 | 002_6_h8 light chain variable region | 80 |

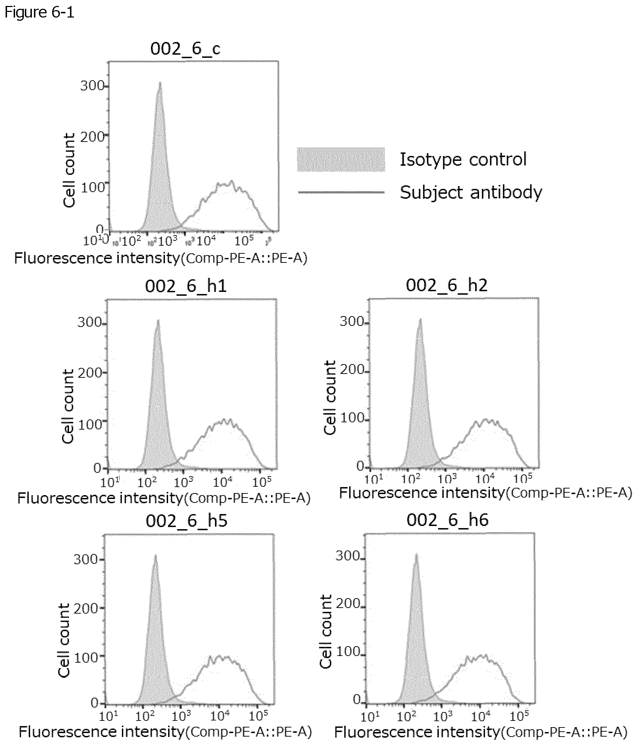

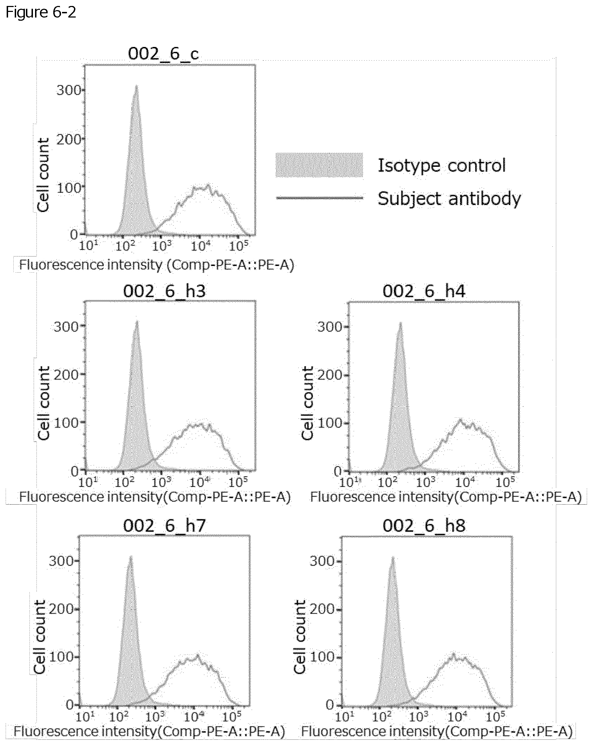

Analysis of the binding activity of each of the obtained humanized antibodies 002_6_h1 to 002_6_h8 to target expressing cells was carried out by FACS analysis using endogeneously CD73 expressing cells (human lung cancer cell line H322). Results are shown in Figure 6. It was confirmed that all humanized antibodies showed the same binding activity as the base chimeric antibody 002_6_c antibody.

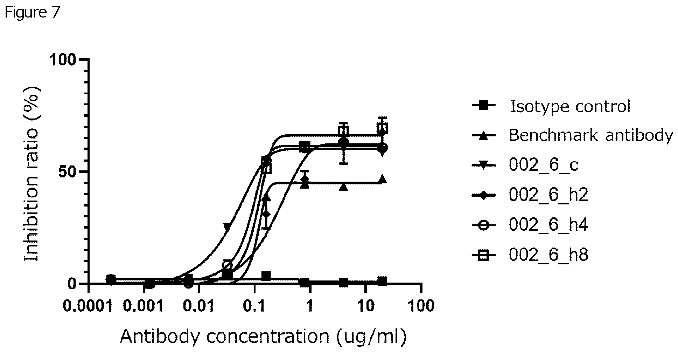

Next, for the 3 antibodies (002_6_h2, 002_6_h4, and 002_6_h8) of the humanized antibodies 002_6_h1 to 002_6_h8, antibody functional screening involving measuring the AMP degradation suppressive function using human lung cancer H322 cells was carried out by the same method as in (1-3). Results are shown in Figure 7.

From the present results, the IC50 value of each of the humanized antibodies for the AMP degradation suppressive function using human lung cancer H322 cells was calculated and found to be as shown in Table 5 below, and it was decided to carry out the subsequent development with 002_6_h4, which had the highest suppressive function among the humanized antibodies, as a lead antibody. This antibody showed a good IC50 value as compared with the benchmark antibody.

[Table 5]

| Name | IC50 value ($\mu$g/ml) |
|---|---|
| 002_6_c | 0.040 |
| 002_6_h2 | 0.236 |
| 002_6_h4 | 0.069 |
| 002_6_h8 | 0.093 |
| Benchmark antibody | 0.091 |

The humanized antibody 002_6_h4 selected as the lead antibody above was compared with existing antibodies (commercially available anti-CD73 antibody (biolegend) and benchmark antibody) and the base chimeric antibody 002_6_c, and the binding activity to various cells was confirmed by flow cytometry.

Analysis of the binding activity to a target expressing cell was carried out by FACS analysis using endogeneously CD73 expressing cells (human breast cancer cell line MDA-MB-231), a human breast cancer cell line MDA-MB-231 cells expressing the CD73 antigen, an HEK293T wild-type cells (derived from a human fetal kidney cell) not expressing the CD73 antigen, and cells obtained by forcibly expressing the CD73 antigen in an HEK293T cell. The CD73 transfected cells was prepared using a lentiviral vector system (Origene). A lentivirus particle carrying the human CD73 gene (NM_002526) and a puromycin resistant gene was prepared and HEK293T cells were infected therewith, then the cells were cultured in the presence of puromycin, and HEK293T cells that became CD73 positive by incorporating the target gene into the genome was recovered and used for binding analysis. The subject antibody or an isotype control antibody was reacted with the cells, then the cells were stained with a fluorescently labeled secondary antibody and subjected to flow cytometry analysis, and the antibody binding activity was evaluated based on the obtained fluorescence intensity.

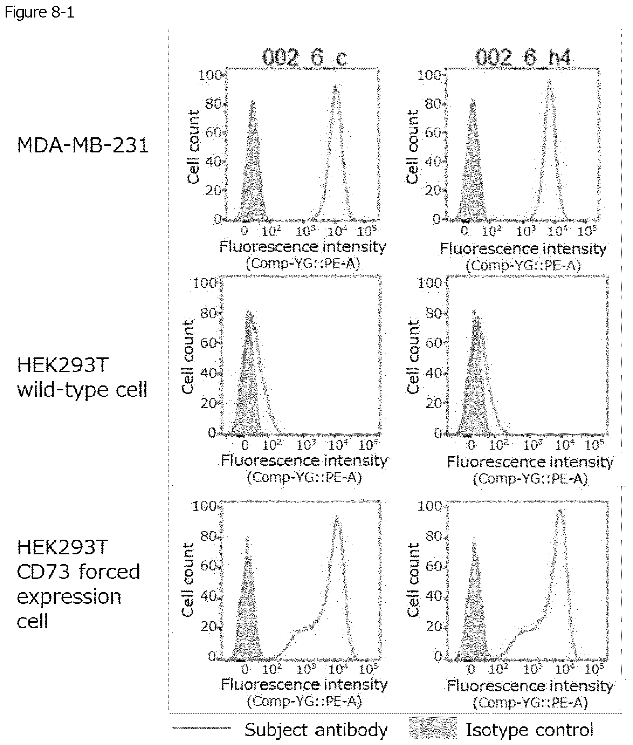

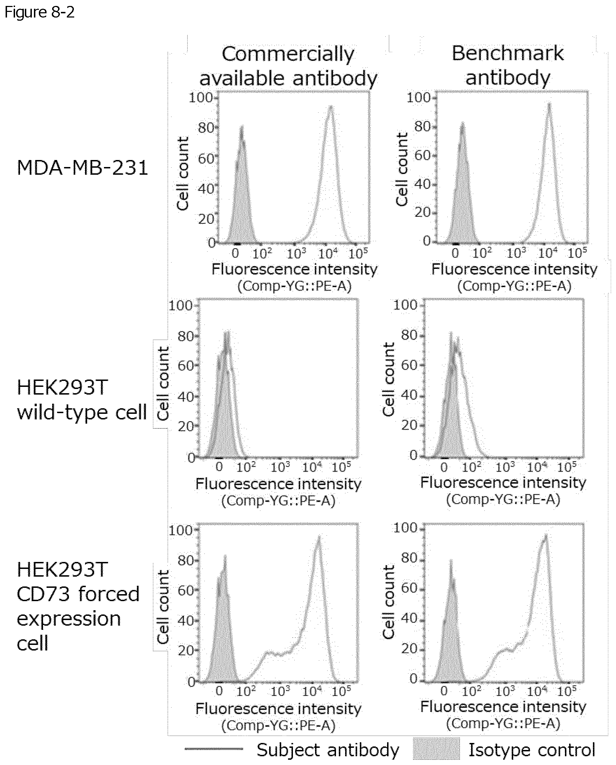

Results are shown in Figure 8. As a result, it was found that both 002_6_c and 002_6_h4 showed the same binding activity to various cells as existing antibodies (commercially available anti-CD73 antibody (biolegend) and benchmark antibody), and the binding activity to the CD73 antigen could be confirmed.

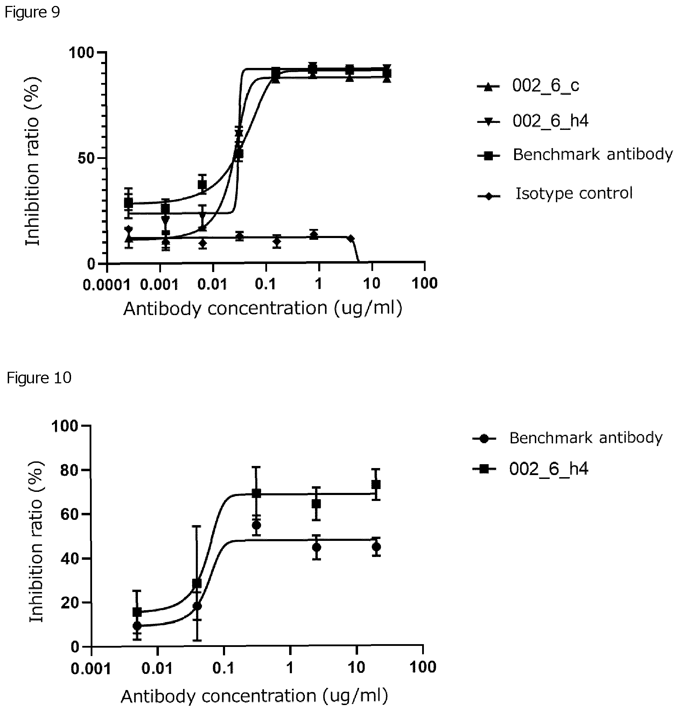

Next, the humanized antibody 002_6_h4 was compared with the existing antibodies (commercially available anti-CD73 antibody (biolegend) and benchmark antibody) and the base chimeric antibody 002_6_c, and antibody function screening involving measuring the AMP degradation suppressive function using human breast cancer MDA-MB-231 cells was carried out by the same method as in (1-3). Results are shown in Figure 9. From the present results, the IC50 value of the humanized antibody for the AMP degradation suppressive function using human breast cancer MDA-MB-231 cells was calculated and found to be as shown in Table 6 below, and together with the results of the test using human lung cancer H322 cells described above, it was shown that the humanized antibody 002_6_h4, like the chimeric antibody 002_6_c, has an AMP degradation suppressive function in a plurality of cancer types.

[Table 6]

| Name | IC50 value (μg/ml) |
|------|------|
| 002_6_c | 0.023 |
| 002_6_h4 | 0.032 |

Example 7: Suppressive function against recombinant antigen

The present Example was carried out for the purpose of confirming the enzyme reaction suppressive function of the anti-CD73 antibody obtained in Example 6 against the recombinant CD73 antigen.

An experiment was carried out by immobilizing the CD73 antigen (terminal His-Tag, self-prepared product) on a 96 well plate for ELISA via an anti-His-Tag antibody, adding the humanized anti-CD73 antibody 002_6_h4 and the substrate AMP (final concentration of 100 μM), incubating the resulting mixture at 37°C for 1 hour, and measuring the reaction of degradation of AMP into adenosine by CD73 in the same manner as in Example 1 (1-3).

The supernatant was recovered, ATP (final concentration of 100 μM) and Cell TiterGlo (Promega, G9243) were added and mixed, and then the chemiluminescence was measured using a microplate reader. Using the principle that AMP inhibits the chemiluminescence due to the reaction between ATP and Cell TiterGlo, the enzyme activity inhibitory effect of the antibodies was evaluated from the amount of residual AMP. The same experiment was carried out using the benchmark antibody as a positive control.

The AMP degradation suppressive function of 002_6_h4 against the recombinant CD73 antigen is shown in Figure 10. As a result, it was suggested that 002_6_h4 showed an AMP degradation suppressive function against the recombinant CD73 antigen and directly suppressed the enzyme.

Example 8: Evaluation of influence on T cell division

The present Example was carried out for the purpose of confirming the proliferation promoting activity of the anti-CD73 antibody obtained in Example 6 on human peripheral blood T cells.

CD4+ T Cell Isolation Kit, human (Miltenyi Biotech, 130-096-533) was used to purify CD4 positive T cells from healthy volunteer-derived peripheral blood mononuclear cells (PBMCs), and CellTrace CFSE cell proliferation kit (ThermoFisher Scientific, #C34554) was used to fluorescently label the cells.

The CD4 positive T cells were activated using Dynabeads™ Human T-Activator CD3/CD28 for T Cell Expansion and Activation (Veritas, DB11131), and then the cells were seeded on a 96 well plate (1 × 10^5 cells/well) and cultured in the presence of the humanized anti-CD73 antibody 002_6_h4 and the substrate AMP (100 μM) for 3 to 7 days. The cells were subjected to flow cytometry analysis to measure the fluorescence intensity, and the ratio of dividing cells was analyzed based on the fluorescence intensity.

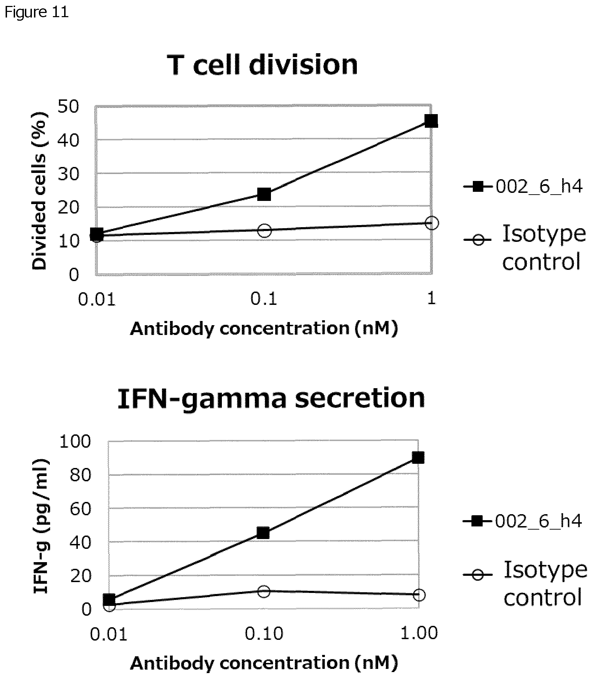

The influence of 002_6_h4 on the division (proliferation) of human peripheral blood T cells is shown in Figure 11. The ratio of dividing T cells increased in an antibody concentration-dependent manner, indicating that the anti-CD73 antibody had the effect of promoting T cell division.

Example 9: Antibody function evaluation (evaluation of concentration-dependency)

The present Example was carried out for the purpose of evaluating the function of the antibodies other than the 002_m antibody obtained in Example 1 (that is, 003_m antibody, 004_m antibody, 005_m antibody, 006_m antibody, 007_m antibody, 008_m antibody, and 009_m antibody).

The antibody function evaluation was carried out by the same method as in the antibody function screening described in Example 1 (1-3). That is, human lung cancer cell line H322 cells or human breast cancer cell line MDA-MB-231 cells (ATCC(registered trademark) HTB-26™) were used as cells. In the present Example, the antibody concentration dependence of the AMP degradation suppressive function was analyzed by measuring the AMP degradation reaction in the same manner as in Example 1, the IC50 value (500 inhibition concentration: here, refers to the antibody concentration that can inhibit 500 of the enzyme function of CD73) was calculated, and the function of the antibody was quantitatively evaluated.

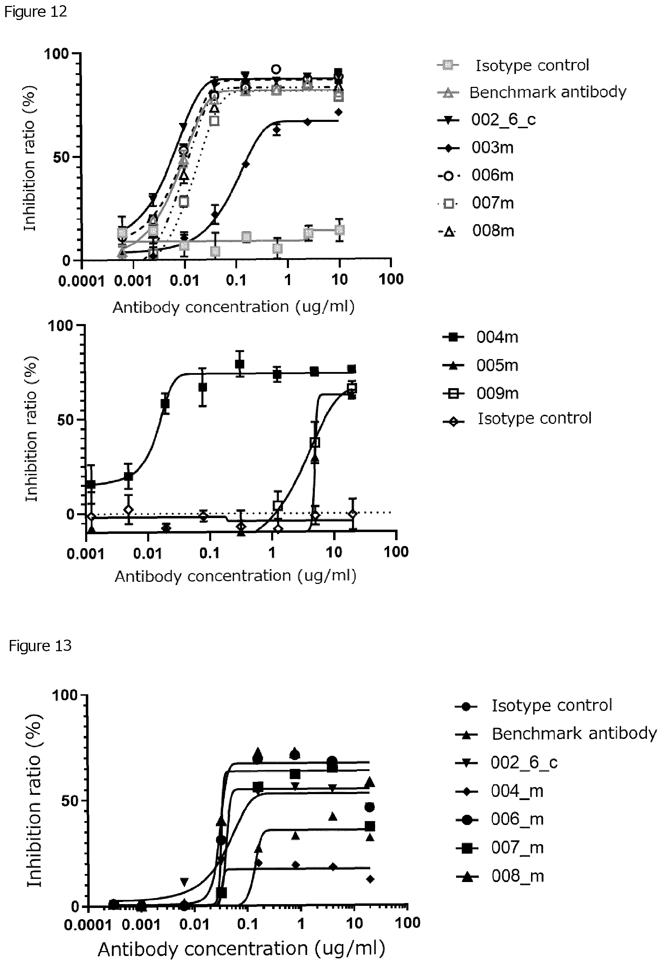

The AMP degradation suppressive function of the 003_m antibody, the 004_m antibody, the 005_m antibody, the 006_m antibody, the 007_m antibody, the 008_m antibody, and the 009_m antibody prepared in Example 1 against the H322 cells is shown in Figure 12, and the AMP degradation suppressive function thereof against the MDA-MB-231 cells is shown in Figure 13. Some of these antibodies, which are mouse antibodies, were found to show an IC50 value lower than or close to that of the benchmark antibody (see Table 7).

[Table 7]

| Sample | IC50 value ($\mu$g/ml) |
|---|---|
| 003_m | 0.088 |
| 004_m | 0.013 |
| 005_m | - |
| 006_m | 0.008 |
| 007_m | 0.012 |
| 008_m | 0.006 |
| 009_m | - |
| Benchmark antibody | 0.007 |

Further, clones considered to have high functionality were selected from the tests shown in Figure 12 and Figure 13 above (004_m, 006_m, 007_m, and 008_m). Then the AMP degradation suppressive function (MDA-MB-231 cells) of these four antibodies is shown. The IC50 values of these clones were good as compared with the benchmark antibody (under clinical development) and were about the same as that of 002_6_c (see Table 8).

[Table 8]

| Sample | IC50 value ($\mu$g/ml) |
|---|---|
| 002_6_c | 0.040 |
| 004_m | 0.032 |
| 006_m | 0.032 |
| 007_m | 0.041 |
| 008_m | 0.031 |
| Benchmark antibody | 0.111 |

Example 10: Antibody gene sequence analysis

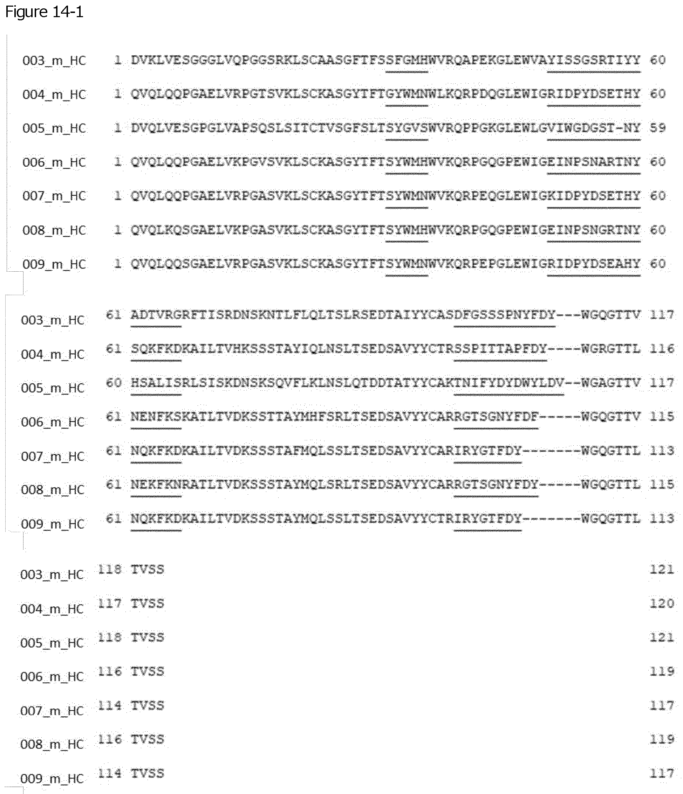

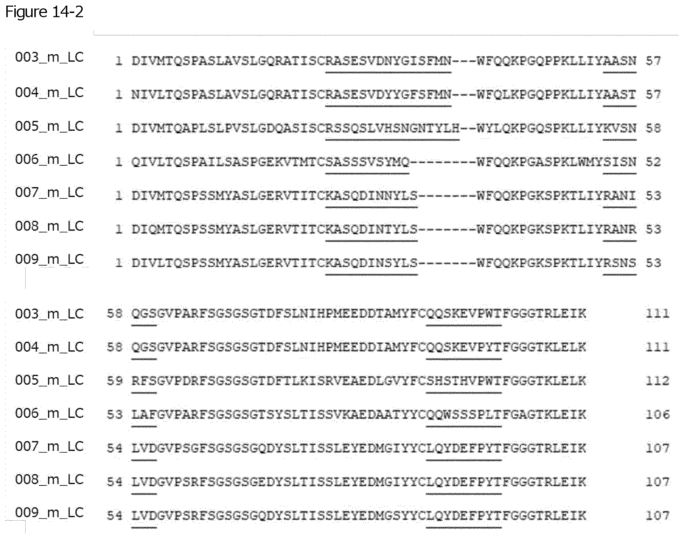

The present Example was carried out for the purpose of determining the amino acid sequences of the 003_m antibody to the 009_m antibody obtained in Example 1.
For clones of the 003_m antibody to the 009_m antibody whose AMP degradation suppressive function was confirmed by functional analysis, a single colony was recovered from an inoculated plate of Escherichia coli transfected with the ligation product prepared in Example 1 (1-1), and plasmid DNA was obtained.
The obtained plasmid DNA was analyzed for the gene sequences defining the heavy chain variable region and the light chain variable region by Sanger sequencing, and the amino acid sequences were determined based on these gene sequences.
The heavy chain variable region amino acid sequences (Figure 14-1) and light chain variable region amino acid sequences (Figure 14-2) of the 003_m antibody, the 004_m antibody, the 005_m antibody, the 006_m antibody, the 007_m antibody, the 008_m antibody, and the 009_m antibody are shown. Each amino acid sequence was assigned SEQ ID NO as shown in Table 9 below.

[Table 9]

| Sequence overview | | SEQ ID NO: | Sequence overview | | SEQ ID NO: |
|---|---|---|---|---|---|
| 003_m heavy chain variable region | | 15 | 006_m light chain variable region | | 40 |
| | CDR1 | 9 | | CDR1 | 36 |

(continued)

| Sequence overview | | SEQ ID NO: | Sequence overview | | SEQ ID NO: |
|---|---|---|---|---|---|
| | CDR2 | 10 | | CDR2 | 37 |
| | CDR3 | 11 | | CDR3 | 38 |
| 003_m light chain variable region | | 16 | 007_m heavy chain variable region | | 47 |
| | CDR1 | 12 | | CDR1 | 41 |
| | CDR2 | 13 | | CDR2 | 42 |
| | CDR3 | 14 | | CDR3 | 43 |
| 004_m heavy chain variable region | | 23 | 007_m light chain variable region | | 48 |
| | CDR1 | 17 | | CDR1 | 44 |
| | CDR2 | 18 | | CDR2 | 45 |
| | CDR3 | 19 | | CDR3 | 46 |
| 004_m light chain variable region | | 24 | 008_m heavy chain variable region | | 55 |
| | CDR1 | 20 | | CDR1 | 49 |
| | CDR2 | 21 | | CDR2 | 50 |
| | CDR3 | 22 | | CDR3 | 51 |
| 005_m heavy chain variable region | | 31 | 008_m light chain variable region | | 56 |
| | CDR1 | 25 | | CDR1 | 52 |
| | CDR2 | 26 | | CDR2 | 53 |
| | CDR3 | 27 | | CDR3 | 54 |
| 005_m light chain variable region | | 32 | 009_m heavy chain variable region | | 63 |
| | CDR1 | 28 | | CDR1 | 57 |
| | CDR2 | 29 | | CDR2 | 58 |
| | CDR3 | 30 | | CDR3 | 59 |
| 006_m heavy chain variable region | | 39 | 009_m light chain variable region | | 64 |
| | CDR1 | 33 | | CDR1 | 60 |
| | CDR2 | 34 | | CDR2 | 61 |
| | CDR3 | 35 | | CDR3 | 62 |

Example 11: Analysis of binding activity to target expressing cells

The present Example was carried out for the purpose of evaluating the binding activity of each antibody of 003_m to 009_m obtained in Example 1.

Analysis of the binding activity to a target expressing cell was carried out by FACS analysis using endogeneously CD73 expressing cells (human lung cancer cell line H322 or human breast cancer cell line MDA-MB-231) or a CD73 transfected cells. The CD73 transfected cells were prepared using a lentiviral vector system (Origene). A lentivirus particle carrying the human CD73 gene (NM_002526) and a puromycin resistant gene was prepared and HEK293T cells were infected therewith, then the cells were cultured in the presence of puromycin, and an HEK293T cells that became CD73 positive by incorporating the target gene into the genome was recovered and used for binding analysis.

As a negative control, HEK293T cells not expressing CD73 was used.

A subject antibody or an isotype control antibody was reacted with the cells, then the cells were stained with a fluorescently labeled secondary antibody and subjected to flow cytometry analysis, and the antibody binding activity was evaluated based on the obtained fluorescence intensity.

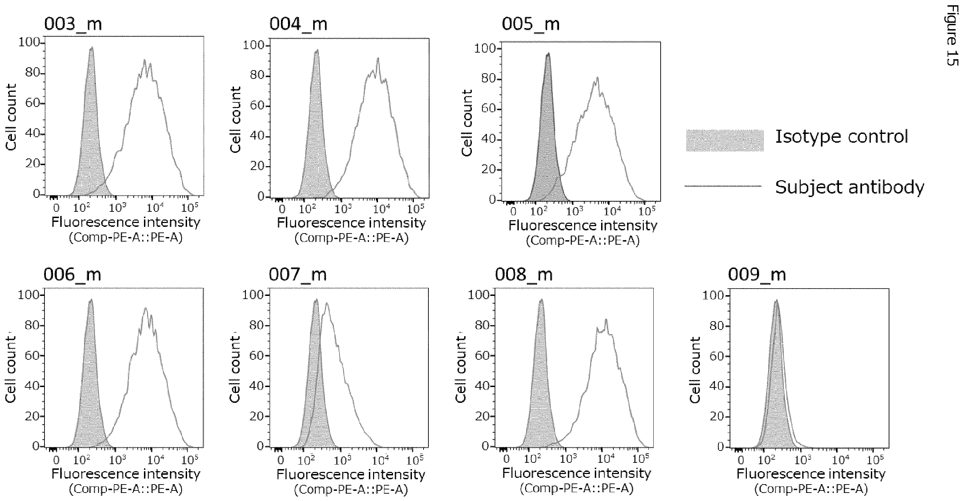

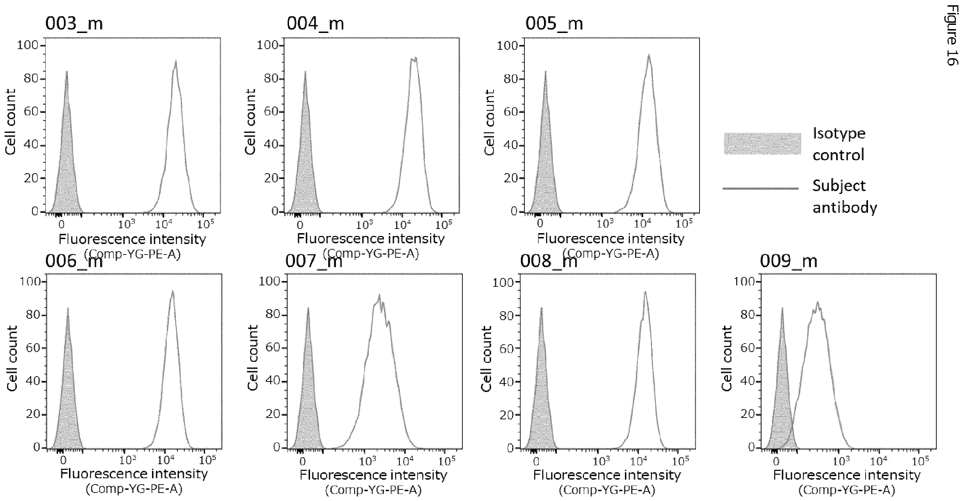

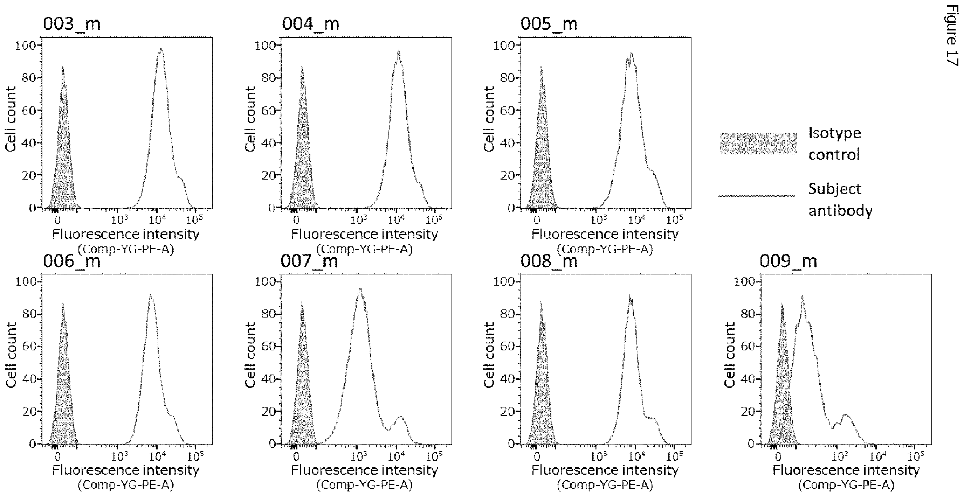

The binding activity of the 003_m antibody to the 009_m antibody to target expressing cells (H322) is shown in Figure 15, the binding activity of the 003_m antibody to the 009_m antibody to target expressing cells (MDA-MB-231) is shown in Figure 16, and the binding activity of the 003_m antibody to the 009_m antibody to a target expressing cell (CD73 transfected HEK-293T cells) is shown in Figure 17. In these Figure 15 to Figure 17, solid lines and gray fills show fluorescence intensity histograms of the subject antibody and the isotype control antibody, respectively.

Example 12: Evaluation of influence on T cell division

The present Example was carried out for the purpose of confirming the proliferation promoting activity of the anti-CD73 antibody obtained in Example 9 on human peripheral blood T cells.

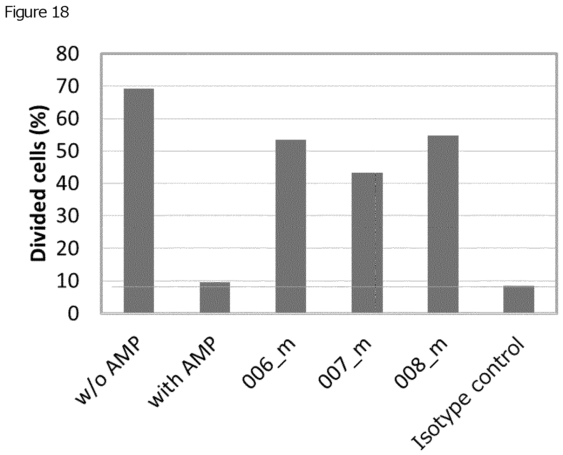

The same method as in Example 8 was adopted, and the dividing cell ratio when the antibody concentration was 1 nM were compared. Results are shown in Figure 18. The dotted line represents the dividing cell ratio in the isotype control antibody.

Specifically, CD4+ T Cell Isolation Kit, human (Miltenyi Biotech, 130-096-533) was used to purify CD4 positive T cells from healthy volunteer-derived peripheral blood mononuclear cells (PBMCs), and CellTrace CFSE cell proliferation kit (ThermoFisher Scientific, #C34554) was used to fluorescently label the cells.

The CD4 positive T cells were activated using Dynabeads™ Human T-Activator CD3/CD28 for T Cell Expansion and Activation (Veritas, DB11131), and then the cells were seeded on a 96 well plate (1 × 10^5 cells/well) and cultured in the presence of an anti-CD73 antibody (006_m antibody, 007_m antibody, or 008_m antibody) and the substrate AMP (100 μM) for 3 to 7 days. The cells were subjected to flow cytometry analysis to measure the fluorescence intensity, and the ratio of dividing cells was analyzed based on the fluorescence intensity.

The influence of a 1 nM anti-CD73 antibody (006_m antibody, 007_m antibody, or 008_m antibody) on the division (proliferation) of a human peripheral blood T cell is shown in Figure 18. In the figure, "w/o AMP" and "with AMP" (100 μM AMP) were incorporated to see the effect of the presence or absence of AMP with no antibody because AMP has a T cell division suppressive effect. On the other hand, in groups in which a humanized anti-CD73 antibody (006_m antibody, 007_m antibody, or 008_m antibody) was added at an amount of 1 nM, 0.1 nM, or 0.01 nM, respectively, the ratio of dividing T cells increased in an antibody concentration-dependent manner (data not shown), indicating that the anti-CD73 antibody had the effect of promoting T cell division.

All of the 003_m antibody to the 009_m antibody promoted cell division more than the isotype control antibody, and were almost equivalent to the benchmark antibody, 002_6_c, and 002_6_h4.

Example 13: Preparation of humanized antibody (2)

The present Example was carried out for the purpose of preparing a humanized antibody based on the 004_m antibody, the 006_m antibody, the 007_m antibody, or the 008_m antibody selected in Example 9 among the antibodies of 003_m to 009_m obtained in Example 1.

The humanized antibody was prepared using the public domain software Tabhu (http://circe.med.unirom-a1.it/tabhu/) as in Example 6. Using the same software, sequences having a property similar in sequence and conformation to the 004_m antibody, the 007_m antibody, or the 008_m antibody were extracted from a human antibody database, 5 sequences for the 004_m antibody, 4 sequences for the 007_m antibody, and 4 sequences for the 008_m antibody were chosen as candidate sequences, and the sequences other than the CDR regions of these antibodies were grafted with the sequences of the CDR regions of the 004_m antibody, the 007_m antibody, and the 008_m antibody, respectively.

For the 006_m antibody, 6 humanized antibody sequence candidates were prepared using commercially available biological software.

Next, backmutation of some amino acid residues of the 004m antibody, the 007m antibody, and the 008m antibody subjected to the grafting to the mouse sequence was carried out. The amino acid residues to be backmutated were selected with an upper limit of 10 amino acid residues for each sequence based on the large structural difference between the humanized antibody and the mouse antibody (higher TubHu score). For details, the method described in Non Patent Literature (Bioinformatics. 2015 Feb 1;31(3):434-5) was followed.

The amino acid sequences of the variable regions of the humanized antibodies (004_h3 antibody, 006_hKB antibody, 007_h1 antibody, and 008_h4 antibody) obtained based on the 004_m antibody, the 006_m antibody, the 007_m antibody, and the 008_m antibody, respectively are shown in Table 10 below. These amino acid sequences were assigned SEQ ID NO: 81 to SEQ ID NO: 88 as shown in Table 10 below.

[Table 10]

| 004_h3_HC (SEQ ID NO: 81) | EVQLVQSGAE VKKPGESLKI SCKASGYTFT GYWMNWLRQM PDKGLEWMGR IDPYDSETHY SQKFKDKVTI TAHKSSSTAY LQWSSLKASD SAIYYCARSS PITTAPFDYW GQGTTVTVSS |
|---|---|
| 004_h3_LC (SEQ ID NO: 82) | DIVMTQSPAS LAVSLGERAT ISCRASESVD YYGFSFMNWY QQKPGQPPKL LIYAASTQGS GVPDRFSGSG SGTDFTLTIS SLQAEDVAVY YCQQSKEVPY TFGGGTKVEI K |
| 006_hKB_HC (SEQ ID NO: 83) | QVQLLQSGAE VKKPGESVKI SCKASGYTFT SYWMHWVKQR PGQGLEWIGE INPSNARTNY NENFKSRFTI TVDKSTSTAY MQFSRLRSDD TAVYYCARRG TSGNYFDFWG QGTMLTVSS |
| 006_hKB_LC (SEQ ID NO: 84) | DIQMTQSPSS LSASLGQRVT ITCKASQDIN TYLSWFQQKP GKPPKTLIYR ANRLVDGVPS RFSGSGSGED YSLTISSLQS EDFGVYYCLQ YDEFPYTFGP GTRLEIK |
| 007_h1_HC (SEQ ID NO: 85) | QVQLVQSGAE VKKPGASVKV SCKASGYTFT SYWMNWVRQA PGQRLEWMGK IDPYDSETHY NQKFKDRVTI TVDTSSSTAY MELSSLRSED TAVYYCARIR YGTFDYWGQG TLVTVSS |
| 007_h1_LC (SEQ ID NO: 86) | DIQMTQSPSS LSASVGDRVT ITCKASQDIN NYLSWYQQKP GKAPKTLIYR ANILVDGVPS GFSGSGSGTD FTLTISSLQY EDMATYYCLQ YDEFPYTFGQ GTKLEIK |
| 008_h4_HC (SEQ ID NO: 87) | QVQLVQSGAE VKKPGASVKV SCKASGYTFT SYWMHWVRQR PGQGLEWMGE INPSNGRTNY NEKFKNRATI TADKSSSTAY MELSSLRSED TAVYYCARRG TSGNYFDYWG QGTLVTVSS |
| 008_h4_LC (SEQ ID NO: 88) | DIQMTQSPSS LSASVGDRVT ITCKASQDIN TYLSWFQQKP GKAPKTLIYR ANRLVDGVPS RFSGSGYGTD FTLTISSLQY EDMATYYCLQ YDEFPYTFGG GTKVEIK |

The humanized antibodies (004_h3 antibody, 006_hKB antibody, 007_h1 antibody, and 008_h4 antibody) obtained were each compared with existing antibodies (commercially available anti-CD73 antibody (biolegend) and benchmark antibody) and the base chimeric antibody 002_6_c, and the binding activity to various cells was confirmed by flow cytometry.

Analysis of the binding activity to human target expressing cells was carried out by FACS analysis using endogeneously CD73 expressing cells (human lung cancer cell line H322). Analysis of the binding activity to a target expressing cell was carried out by FACS analysis using endogeneously CD73 expressing cells (human breast cancer cell line MDA-MB-231), human breast cancer cell line MDA-MB-231 cells expressing the CD73 antigen, HEK293T wild-type cells (derived from a human fetal kidney cell) not expressing the CD73 antigen, and cells obtained by forcibly expressing the CD73 antigen in HEK293T cells. The CD73 transfected cells were prepared using a lentiviral vector system (Origene). A lentivirus particle carrying the human CD73 gene (NM_002526) or the rhesus CD73 gene (XM_001086989.2) (the amino acid sequence is the same as that of the cynomolgus CD73), and a puromycin resistant gene was prepared and HEK293T cells were infected therewith, then the cells were cultured in the presence of puromycin, and HEK293T cells that became CD73 positive by incorporating the target gene into the genome was recovered and used for binding analysis. A subject antibody or an isotype control antibody was reacted with the cells, then the cells were stained with a fluorescently labeled secondary antibody and subjected to flow cytometry analysis, and the antibody binding activity was evaluated based on the obtained fluorescence intensity.

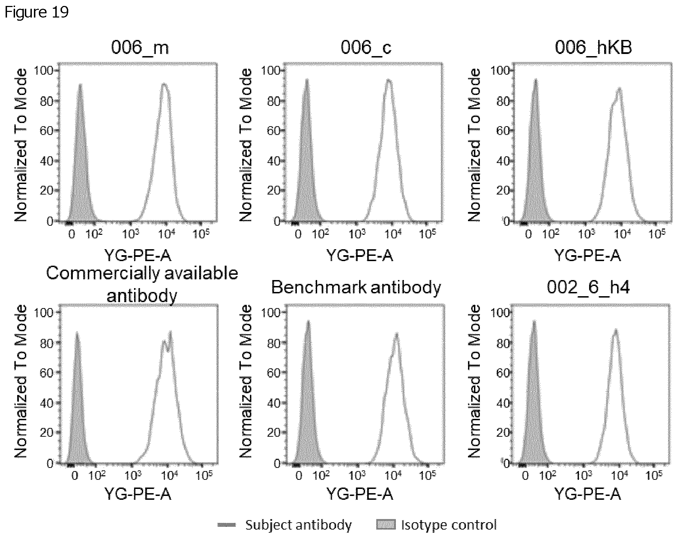

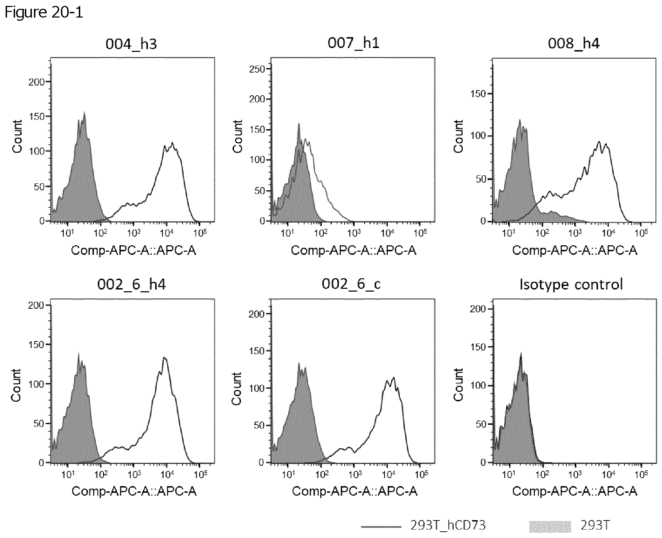

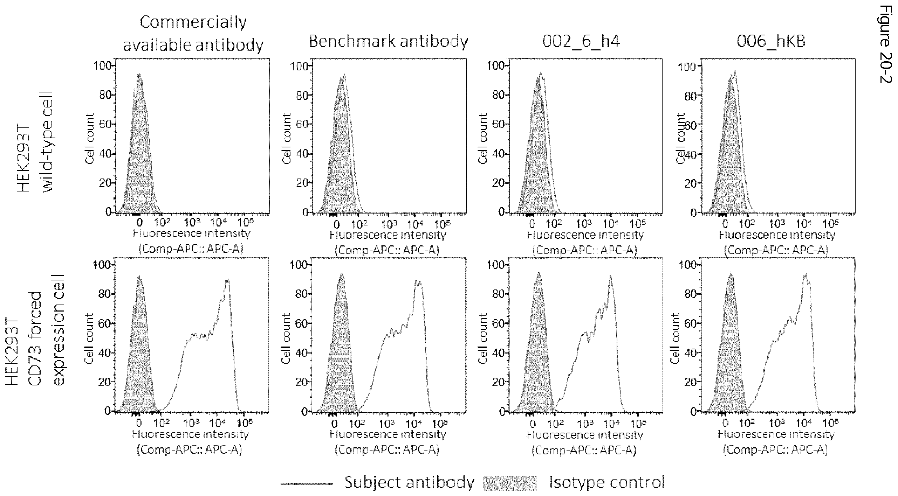

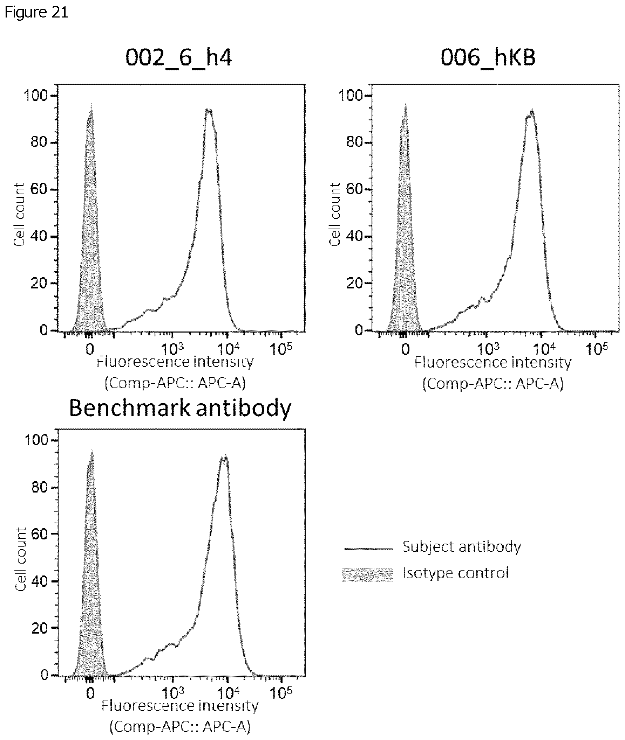

Results are shown in Figure 19 to Figure 21. Among these, Figure 19 and Figure 20 are diagrams confirming the binding activity of the antibodies to the cells expressing the human CD73 gene, and Figure 21 is diagrams confirming the binding activity of the antibodies to the cells expressing the rhesus CD73 gene. It was confirmed that the 004_h3 antibody, the 006_hKB antibody, and the 008_h4 antibody among the humanized antibodies studied showed the same binding activity to the human CD73 antigen as did the 002_6_h4 antibody to be compared therewith, and the binding activity to the CD73 antigen was able to be confirmed. In addition, it was confirmed that the 006_hKB antibody among these showed the same binding activity to the rhesus CD73 antigen (cynomolgus CD73 antigen) as did the 002_6_h4 antibody, indicating that when the effect of the antibody of the present invention is confirmed by an animal experiment in the future, a rhesus monkey or a cynomolgus monkey can be used to carry out a study.

Example 14: Suppressive function against recombinant antigen

The present Example was carried out for the purpose of confirming the enzyme reaction suppressive function of the humanized anti-CD73 antibody obtained in Example 13 against the recombinant CD73 antigen.

The antibody function evaluation was carried out by the same method as in the antibody function screening described in Example 1 (1-3) using the 004_h3 antibody, the 006_hKB antibody, the 007_h1 antibody, and the 008_h4 antibody as subject antibodies, and using the 002_6_c antibody, the 002_6_h4 antibody, the isotype control antibody, and the benchmark antibody (MEDI9447 antibody) as controls. That is, human breast cancer cell line MDA-MB-231 cells (ATCC(registered trademark) HTB-26™) as cells were seeded on a 96 well plate and incubated overnight at 37°C. The next day, the cells were washed with L-15 medium (FUJIFILM Wako Pure Chemical Corporation, 128-06075), then a purified antibody was added, followed by incubation at 37°C for 1 hour. In the present Example, as in Example 1 (1-3), AMP (Sigma, A1752), which is a substrate of CD73, was added such that a final concentration of 200 $\mu$M was reached, and the resulting mixture was incubated at 37°C for 4.5 hours to measure the reaction of degradation of AMP into adenosine by CD73, and thereby the antibody concentration dependence of the AMP degradation suppressive function was analyzed. A degradation reaction was carried out. After the reaction, the supernatant was recovered, ATP (Sigma, A2383, final concentration of 10 $\mu$M) and Cell TiterGlo (Promega, G9243) were added and mixed, and then the chemiluminescence was measured using a microplate reader.

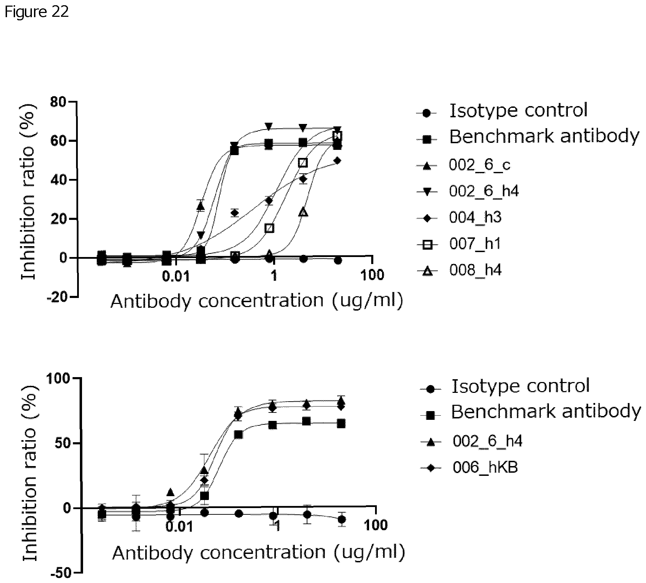

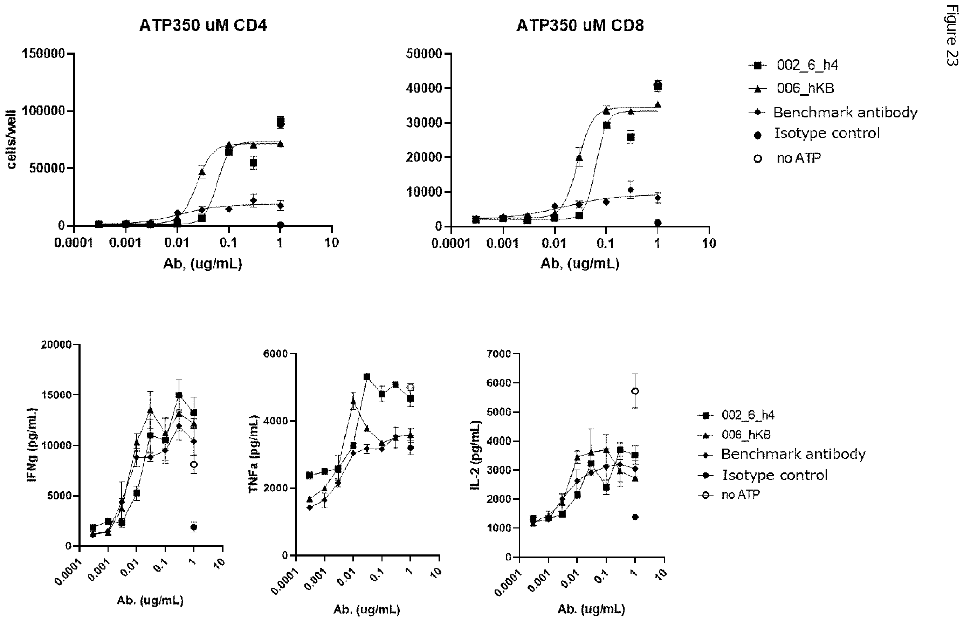

The AMP degradation suppressive function of the 004_h3 antibody, the 006_hKB antibody, the 007_h1 antibody, and the 008_h4 antibody is shown in Figure 22. All of these antibodies were shown to have the function of suppressing AMP degradation by CD73 present on the cell surface (Figure 22 and Figure 23), and among these antibodies, particularly the 006_hKB antibody, like 002_6_h4, was shown to have an inhibitory activity equal to or higher than that of the benchmark antibody.

Example 15: Evaluation of influence on T cell division

The present Example was carried out for the purpose of confirming the proliferation promoting activity of the anti-CD73 antibody obtained in Example 13 and showing a particularly high effect in Example 14 on human peripheral blood T cells.

To a healthy volunteer derived peripheral blood mononuclear cells (PBMCs), any of various antibodies (006_hKB antibody, 002_6_h4 antibody, benchmark antibody (MEDI9447 antibody), and isotype control antibody) having a final concentration of 0.0005 to 1 $\mu$g/ml, and the resulting mixture was incubated for 30 minutes at room temperature. Subsequently, ATP having a final concentration of 350 $\mu$M and Dynabeads™ Human T-Activator CD3/CD28 for T Cell Expansion and Activation (Veritas, DB11131) were added, and the resulting mixture was incubated under conditions of 37°C and 5% $CO_2$ for 96 hours. After completion of the culture, the cells were stained using T cell marker staining antibodies (BioLegend, anti-CD3: UCHT1, anti-CD4: RPA-T4, anti-CD8a: HIT8a). Cell counting beads were added to the stained cells and analyzed using a flow cytometer to calculate the numbers of CD4 and CD8 positive cells per well.

In addition, the concentrations of IFN$\gamma$, TNF$\alpha$, and IL-2 in the culture supernatant recovered were measured by the Luminex method using various antibodies [capture antibodies = IFN$\gamma$: M7004 (Thermo), TNF$\alpha$: MAB602 (R&D Systems), IL-2: MAB610; detection antibodies = IFN$\gamma$: M701B (Thermo), IL-2: BAF202 (R&D Systems), TNF$\alpha$: BAF210 (R&D Systems)].

Results of the evaluation of the influence of the antibodies on T cell division are shown in Figure 23. When compared with the 002_6_h4 antibody, which was found to show a good activity in Example 8, the 006_hKB antibody was shown to have a T cell division promoting effect (upper row of Figure 23) as high as the 002_6_h4 antibody on both the CD4 positive T cells and the CD8 positive T cells. In addition, it was also confirmed that high concentrations of IFN$\gamma$, TNF$\alpha$, and IL-2 were released into the culture supernatant from the cells to which the 006_hKB antibody was administered (lower row of Figure 23).

Example 16: Evaluation of intracellular uptake of antibody

The present Example was carried out for the purpose of evaluating intracellular uptake of the anti-CD73 antibody obtained in Example 6 and the anti-CD73 antibody obtained in Example 13 and showing a particularly high effect in Example 14.

The human breast cancer cells MDA-MB-231 (1 × 10^5 cells) were seeded on a chamber slide (Thermo Fisher Scientific, 154534PK), various antibody solutions (002_6_h4, 006_hKB, MEDI9447) having 10 μg/ml were added, and the resulting mixture was incubated at 37°C for 15 minutes or 19 hours. Samples before and after incubation were fixed and permeabilized (Thermo Fisher Scientific, FIX & PERM Fixation and Permeabilization Kit, GAS003) and stained with Anti-human IgG Alexa Fluor 594 (Thermo Fisher Scientific, A-11014). The distribution of an antibody was observed using a fluorescence microscope (Thermo Fisher Scientific, EVOS FLoid Imaging System, 4471136) to determine the presence or absence of the intracellular uptake of the antibody.

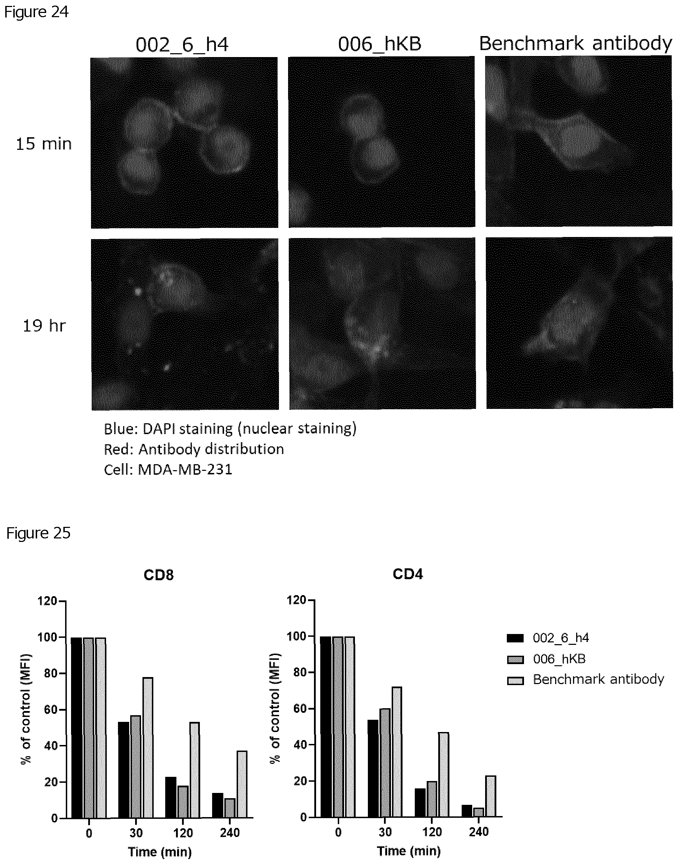

The intracellular uptake of 002_6_h4 and 006_hKB is shown in Figure 24. For each of both 002_6_h4 and 006_hKB, the antibody was distributed on the cell surface 15 minutes after incubation (upper row of Figure 24), whereas the antibody was accumulated in the cells 19 hours after incubation (lower row of Figure 24), indicating that the intracellular uptake of the antibody occurred. The present result suggests that the uptake of the anti-CD73 antibody may induce the disappearance of the antigen from the cell surface.

Such internalization of an antibody into T cells was quantified with time. Healthy volunteer derived peripheral blood mononuclear cells (PBMCs) were stimulated with Dynabeads™ Human T-Activator CD3/CD28 for T Cell Expansion and Activation (Veritas, DB11131) for 6 days, then the cells were recovered, various antibodies (002_6_h4 antibody, 006_hKB antibody, benchmark antibody (MEDI9447 antibody), isotype control antibody) having 10 μg/ml were added, and the resulting cells were incubated on ice for 30 minutes.

The cells were cleaned and then suspended in a FACS buffer (PBS, 0.5% BSA, 2 mM EDTA) and incubated at 37°C for up to 240 minutes, cell samples were sampled at 30, 120, and 240 minutes during the incubation, each sample was subjected to flow cytometry analysis after staining the cells with a fluorescently labeled secondary antibody (APC anti-human IgG Fc) (BioLegend, 409306), and the internalization of the antibodies was evaluated based on the obtained fluorescence intensity. The internalization ratio of the antibodies was calculated by the following expression.

```
% Control = (anti-CD73 antibody (X minutes) MFI - isotype control
(X minutes) MFI)/(anti-CD73 antibody (0 minutes) MFI - isotype
control (0 minutes) MFI) × 100
```

Results are shown in Figure 25. 002_6_h4 and 006_hKB had a higher ratio of internalization than the benchmark antibody at each measurement time. The present result also suggests that the uptake of the anti-CD73 antibody may induce the disappearance of the antigen from the cell surface.

The internalization into cell surface CD73 in cancer cells treated with the antibody of the present invention was quantified with time. Human breast cancer cells MDA-MB-231 and human melanoma cells HT-144 were suspended in a medium supplemented with no fetal bovine serum of L-15 medium for MDA-MB-231 (FUJIFILM Wako Pure Chemical Corporation, 128-06075) and McCoy's 5A (Modified) (Gibco, 16600882) for HT-144, respectively, then various antibodies (002_6_h4 antibody, 006_hKB antibody, benchmark antibody (MEDI9447 antibody), isotype control antibody) having 10 μg/ml were added, and the resulting suspensions were incubated at 37°C for 1 hour, 3 hours, 6 hours, 22 hours, and 48 hours.

Various antibodies were added again at 10 μg/mL for reaction, the cells were cleaned and then stained with a fluorescently labeled secondary antibody and subjected to flow cytometry analysis, and the internalization of the antigen was evaluated based on the obtained fluorescence intensity. The internalization ratio of the antigen was calculated by the following expression.

```
% CD73 Ag = (anti-CD73 antibody (X minutes) MFI - isotype control
(X minutes) MFI)/(anti-CD73 antibody (0 minutes) MFI - isotype
control (0 minutes) MFI) × 100
```

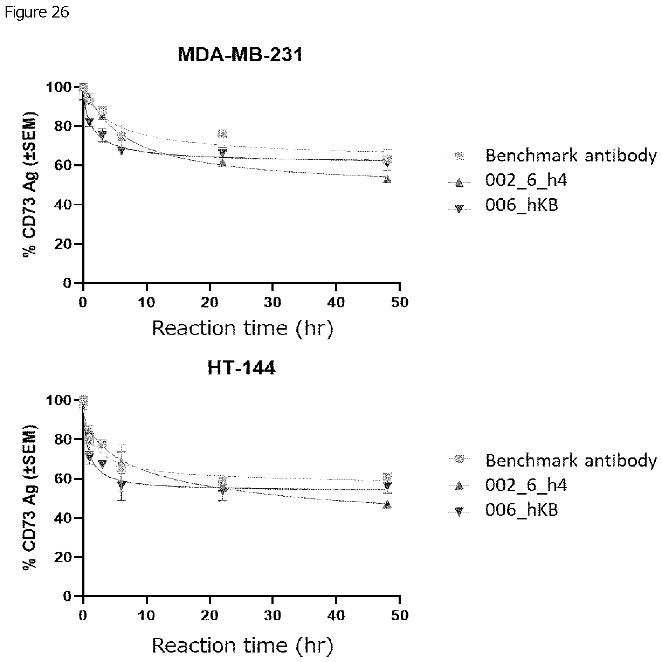

Results are shown in Figure 26. All of 002_6_h4, 006_hKB, and the benchmark antibody reduced the expression level of CD73 on the cancer cell surface with time by incubation thereof with the cancer cells. It was suggested that this was based on the anti-CD73 antibody bound to CD73 being internalized together with the antigen to thereby reduce the expression level of CD73 on the cell surface.

Example 17: Measurement of KD value

The present Example was carried out for the purpose of evaluating the dissociation constant (KD value), which was an index of the binding affinity of the anti-CD73 antibody obtained in Example 6 and the anti-CD73 antibody obtained in Example 13 and showing a particularly high effect in Example 14 to the CD73 antigen.

The KD value of an antibody bound to a target antigen was measured using Biacore 8K (GE Healthcare). Each antibody of the 002_6_h4 antibody and the 006_hKB antibody was immobilized on a sensor chip (CM5) using Human Antibody Capture Kit Type 2 (Cytiva, 26-2346-00). Solutions of the human CD73 antigen diluted serially from $5 \times 10^{-9}$ M in 4 steps were prepared and subjected to multicycle kinetic analysis. With a 1:1 binding model, the KD values were as shown in Table 11 below.

[Table 11]

| Antibody | KD value for human CD73 | KD value for cynomolgus CD73 |
|---|---|---|
| 002_6_h4 antibody | $2.04 \times 10^{-10}$ M | $2.73 \times 10^{-10}$ M |
| 006_hKB antibody | $4.26 \times 10^{-10}$ M | $4.57 \times 10^{-10}$ M |

Example 18: Immunologically humanized mouse test

The present Example was carried out for the purpose of confirming the antitumor effect of the anti-CD73 antibody obtained in Example 6 and the anti-CD73 antibody obtained in Example 13 and showing a particularly high effect in Example 14 in the body of a human CD34 positive cell transplanted mouse.

The human breast carcinoma cell line MDA-MB-231 was transplanted into the mammary fat pad of NSG mice (hu-NSG, JACKSON laboratories) into which human CD34 positive cells prepared from a plurality of healthy donors were transplanted to produce a human breast carcinoma cell-bearing mouse model. When the human breast carcinoma cells transplanted into this mouse model proliferated to 100 mm$^3$, the 002_6_h4 antibody or the isotype control antibody was intraperitoneally administered at a dose of 20 mg/kg once every 4 days a total of 7 times (indicated as "Q4Dx7"). The day when the administration was started was defined as day 0, and observation was carried out until day 34.

After completion of the observation period, the tumor was removed and formed into a cell suspension using Gentle MACS (Miltenyi), then Human (hu)CD45 AF700 clone HI30 (BioLegend), huCD3 APCCy7 clone HITa (BioLegend), huCD4 PECy7 clone SK3 (BioLegend), and huCD8 FITC clone SK1 (BioLegend) were used to stain the cells, and the amounts of CD45+, CD3+, and CD8+ T cells and CD45+, CD3+, and CD4+ T cells infiltrating the tumor were measured by flow cytometry.

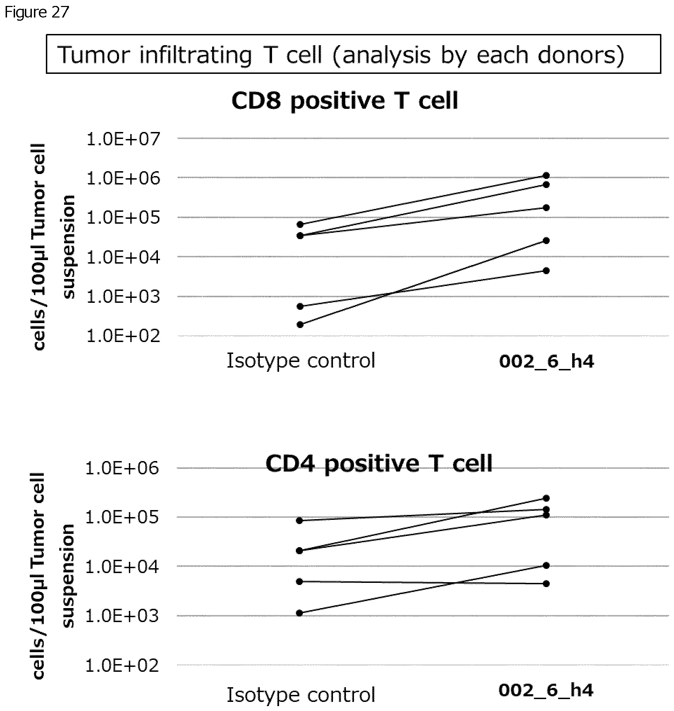

Results of measuring the number of tumor-infiltrating T cells in the mouse body on day 34 after administration of the 002_6_h4 antibody or the isotype control antibody are shown in Figure 27. The administration of the 002_6_h4 antibody showed a tendency for tumor-infiltrating T cells to increase as compared with the case of the administration of the isotype control antibody, suggesting that the anti-CD73 antibody has a tumor immunity effect.

In the present Example, it was further confirmed whether or not the developed antibodies enhance the cytolytic activity of T cells against cancer cells, and if so, how much the developed antibodies do so, in comparison with the benchmark antibody.

Human lung cancer cells NCI-H292 or HCC827 to which RFP protein was introduced were seeded on a 96 well plate and cultured overnight. The medium was removed the next day, then IL-2 (final concentration of 30 IU) suspended in X-VIVO-15 medium (Lonza, 04-418Q) and various antibodies were added, and the resulting mixture was incubated at 37°C for 30 minutes. After that, healthy volunteer PBMCs stimulated with Dynabeads™ Human T-Activator CD3/CD28 for T Cell Expansion and Activation (Veritas, DB11131) for 10 days to proliferate T cells were added at an E/T ratio of 2 and incubated at 37°C for 30 minutes, then ATP having a final concentration of 500 μM was added to NCI-H292 and ATP having a final concentration of 300 μM was added to HCC827, and changes with time in cancer cell proliferation were observed under conditions of 37°C and 5% $CO_2$ using IncuCyte ZOOM System (Sartorius).

As controls, in addition to the isotype control antibody and the benchmark antibody (MEDI9447), an anti-PD-1 antibody (pembrolizumab, Keytruda, MSD) and an anti-PD-L1 antibody (atezolizumab), which act to interfere with the interaction between the PD-1 antigen of cancer cells and the PD-L1 antigen of T cells to inhibit T cell suppression and activate T cells, were used.

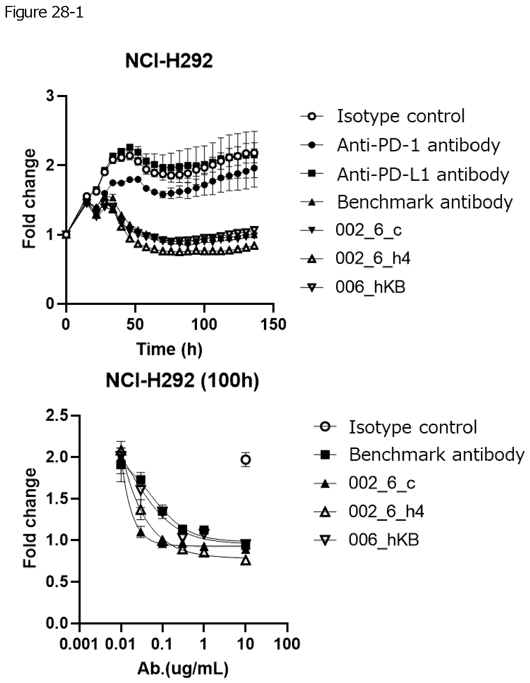

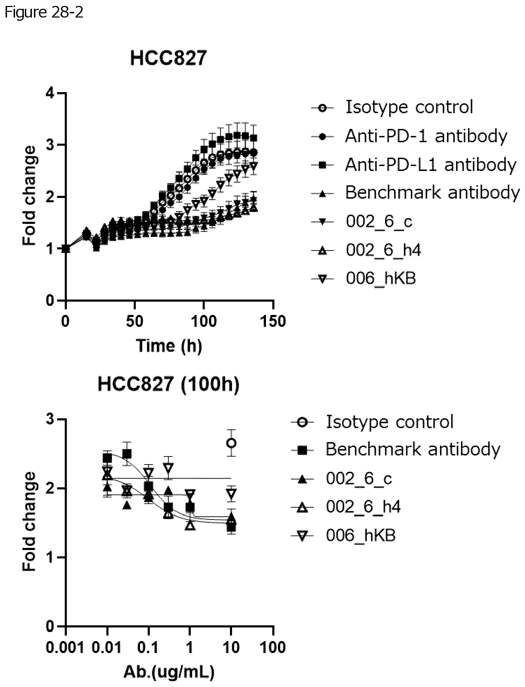

Results are shown in Figure 28. The developed antibodies (in the present Example, 002_6_h4 and 006_hKB were used as examples, but the same also applies to the other developed antibodies of the present invention) had almost no effect of releasing T cell suppression even when the anti-PD-1 antibody and the anti-PD-1 antibody were allowed to act, and enhanced the suppressive effect of T cells on the proliferation of cancer cells under a condition that was unable to stop the proliferation of cancer cells.

In the present Example, it was further investigated by an animal experiment whether or not 002_6_h4 was able to enhance the antitumor effect of the human immune system reproduced in a simulated manner in mice. At that time, it was also confirmed whether an additional effect was able to be seen by combined administration with an anti-PD-1 antibody (pembrolizumab) whose effect had been proven clinically.

The human breast carcinoma cell line MDA-MB-231 was transplanted into the mammary fat pad of NSG mice (hu-NSG, JACKSON laboratories) into which human CD34 positive cells prepared from a plurality of healthy donors were transplanted (hereinafter referred to as "immunologically humanized mice") to produce a human breast carcinoma-bearing mouse model. In this mouse model, when the transplanted human breast carcinoma cells proliferated to 100 $mm^3$, intraperitoneal administration of the 002_6_h4 antibody or the isotype control antibody at a dose of 20 mg/kg once every 4 days a total of 7 times (indicated as "Q4Dx7") in combination with a checkpoint inhibitor (the anti-PD-1 antibody pembrolizumab, Keytruda, MSD) or the isotype control antibody at a dose of 10 mg/kg for the first time only and 5 mg/kg for the second and subsequent times once every 5 days a total of 6 times (indicated as "Q5Dx6") were carried out. The day when the administration was started was defined as day 0, and the tumor size was measured twice a week until day 34. The administration of the antibodies is as follows:

Group 1 = Isotype control antibody only administration group
Group 2 = 002_6_h4 antibody and isotype control antibody administration group
Group 3 = Checkpoint inhibitor (pembrolizumab) and isotype control antibody administration group
Group 4 = 002_6_h4 antibody and checkpoint inhibitor (pembrolizumab) combined administration group.

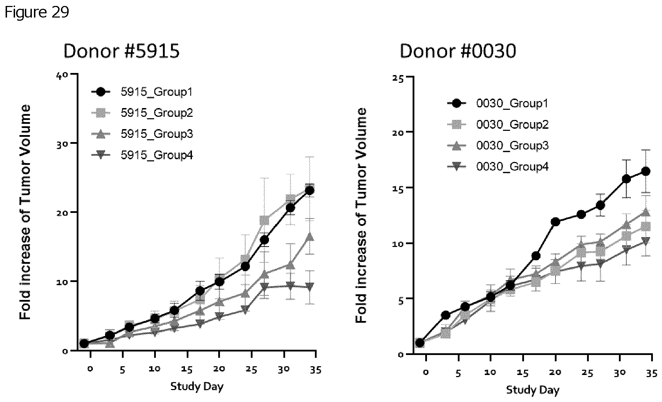

The average values and standard errors of the increase rates of tumor volume from day 0 to day 34 in each administration group in typical donors are shown in Figure 29. In both donor #5910 and donor #0030, the average tumor volume increase rate was lower in the combined administration group (Group 4) than in the control group (Group 1) and the checkpoint inhibitor administration group (Group 3).

Further, in donor #0030, the average tumor volume increase rate was lower in the 002_6_h4 antibody administration group (Group 2) than in the control group (Group 1), suggesting that the anti-CD73 antibody had an antitumor action in immunologically humanized mice when used alone or in combination with a checkpoint inhibitor (pembrolizumab).

Example 19: Immunodeficient mouse test

The present Example was carried out for the purpose of confirming the antitumor effect of the anti-CD73 antibody obtained in Example 6 in the body of immunodeficient mice.

The human breast carcinoma cell line MDA-MB-231 was transplanted into the mammary fat pad of NSG mice (JACKSON laboratories). When the human breast carcinoma cells transplanted proliferated to 100 $mm^3$, the 002_6_h4 antibody or the isotype control antibody was intraperitoneally administered at each dose once every 4 days a total of 7 times (Q4Dx7) in the group configuration shown in Table 12 below. The day when the administration was started was defined as day 0, and the tumor size was measured twice a week with the observation period being up to day 35.

[Table 12]

| Test drug | Administration dose (mg/kg) | Route of administration | Administration frequency |
|---|---|---|---|
| 002_6_h4 | 20 | IP | Q4Dx7 |
| 002_6_h4 | 10 | | |
| 002_6_h4 | 5 | | |
| Isotype control | 20 | | |

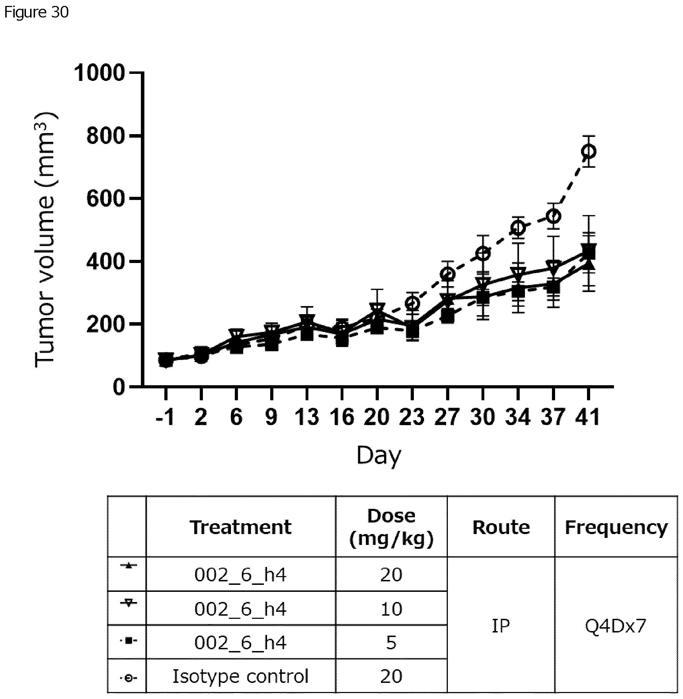

Results of changes in the volume of a tumor mass in the body of the immunodeficient mice are shown in Figure 30. The 002_6_h4 antibody was shown to have a suppressive effect on tumor proliferation as compared with the isotype control.

Example 20: Antitumor effect (knock-in model)

The present Example was carried out for the purpose of confirming the antitumor effect of the anti-CD73 antibody obtained in Example 6 in the body of a knock-in mouse expressing human CD73.

$5 \times 10^5$ cells of a colorectal cancer cell line (Biocytogen, MC38-hCD73) deficient in mouse CD73 and expressing human CD73 were transplanted subcutaneously in knock-in mice (Biocytogen, B-hCD73 mice) expressing human CD73 in place of mouse CD73. When the transplanted MC38-hCD73 proliferated to 50 to 80 mm3, intraperitoneal administration of the 002_6_h4 antibody or the isotype control antibody at a dose of 10 mg/kg twice a week a total of 7 times (indicated as "BIWx7") in combination with the anti-mouse PD-1 antibody clone RMP1-14 (Bio X Cell, BE0146) or a rat IgG2a isotype control antibody (Bio X Cell, BE0089) at a dose of 3 mg/kg once a week (indicated as "QWx4") was carried out. While measuring the tumor size every 3 or 4 days, the day when the administration was started was defined as day 0, and observation was carried out until day 18. The administration of the antibodies is as follows:

Group 1 = Isotype control antibody only administration group
Group 2 = 002_6_h4 antibody and isotype control antibody administration group
Group 3 = Checkpoint inhibitor (anti-mouse PD-1 antibody) and isotype control antibody administration group
Group 4 = Group in which combined administration of the 002_6_h4 antibody and the checkpoint inhibitor (anti-mouse PD-1 antibody) was carried out.

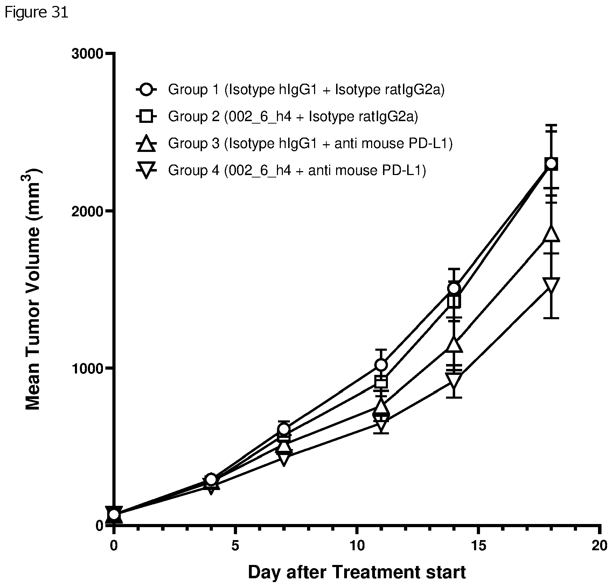

The changes in average tumor volume from day 0 to day 18 as the average values and standard errors in each administration group are shown in Figure 31. The average tumor volume was lower in the combined administration group (Group 4) than in the control group (Group 1) and the checkpoint inhibitor administration group (Group 3), suggesting that the anti-CD73 antibody had an antitumor action in mice expressing human CD73 when used in combination with a checkpoint inhibitor.

Industrial Applicability

The antibody or human-type antibody derivative obtained by the present invention suppresses the function of CD73 expressed in cancer cells or immune cells and releases the exhaustion of the immune cell (particularly T cells) and thereby activates the immune cell and exerts a tumor proliferation suppressive effect and a cancer treatment effect.
The present invention can be widely used as a therapeutic and/or diagnostic agent for a cancer. The antibody according to the present invention has the effect of releasing the exhaustion of immune cells, and thus is expected to be particularly effective in a combination therapy with other immunotherapies.

## Claims

1. An antibody or a human-type antibody derivative that has a binding activity to a CD73 antigen and activates T cells having a toxic activity to cancer cells, comprising complementarity determining regions of any combination of heavy and light chains selected from the group consisting of:

(1) complementarity determining regions of a heavy chain, CDR1 ($DX_1NMD$ (wherein $X_1$ is C or S), SEQ ID No.: 1), CDR2 (DINPNNGGTIYNQKFKG, SEQ ID No.: 2), and CDR3 (TNWDYAMDY, SEQ ID No.: 3) and complementarity determining regions of a light chain, CDR1 ($KASQDINSX_2LS$ (wherein $X_2$ is N, D, or Q), SEQ ID No.: 4), CDR2 (RANRLID, SEQ ID No.: 5), and CDR3 ($X_3QYDVFPRT$ (wherein $X_3$ is L or Q), SEQ ID No.: 6);
(2) complementarity determining regions of a heavy chain, CDR1 (SFGMH, SEQ ID No.: 9), CDR2 (YISSGSRTIYYADTVRG, SEQ ID No.: 10), and CDR3 (DFGSSSPNYFDY, SEQ ID No.: 11), and complementarity determining regions of a light chain, CDR1 (RASESVDNYGISFMN, SEQ ID No.: 12), CDR2 (AASNQGS, SEQ ID No.: 13), and CDR3 (QQSKEVPWT, SEQ ID No.: 14);
(3) complementarity determining regions of a heavy chain, CDR1 (GYWMN, SEQ ID No.: 17), CDR2 (RIDPYDSETHYSQKFKD, SEQ ID No.: 18), and CDR3 (SSPITTAPFDY, SEQ ID No.: 19), and complementarity determining regions of a light chain, CDR1 (RASESVDYYGFSFMN, SEQ ID No.: 20), CDR2 (AASTQGS, SEQ ID No.: 21), and CDR3 (QQSKEVPYT, SEQ ID No.: 22);
(4) complementarity determining regions of a heavy chain, CDR1 (SYGVS, SEQ ID No.: 25), CDR2 (VIWGDGSTNYHSALIS, SEQ ID No.: 26), and CDR3 (TNIFYDYDWYLDV, SEQ ID No.: 27), and complementarity determining regions of a light chain, CDR1 (RSSQSLVHSNGNTYLH, SEQ ID No.: 28), CDR2 (KVSNRFS, SEQ ID No.: 29), and CDR3 (SHSTHVPWT, SEQ ID No.: 30);
(5) complementarity determining regions of a heavy chain, CDR1 (SYWMH, SEQ ID No.: 33), CDR2 (EINPSNARTNYNENFKS, SEQ ID No.: 34), and CDR3 (RGTSGNYFDF, SEQ ID No.: 35), and complementarity determining regions of a light chain, CDR1 (KASQDINTYLS, SEQ ID No.: 36), CDR2 (RANRLVD, SEQ ID No.: 37), and CDR3 (LQYDEFPYT, SEQ ID No.: 38);
(6) complementarity determining regions of a heavy chain, CDR1 (SYWMN, SEQ ID No.: 41), CDR2 (KIDPYDSETHYNQKFKD, SEQ ID No.: 42), and CDR3 (IRYGTFDY, SEQ ID No.: 43), and complementarity determining regions of a light chain, CDR1 (KASQDINNYLS, SEQ ID No.: 44), CDR2 (RANILVD, SEQ ID No.: 45), and CDR3 (LQYDEFPYT, SEQ ID No.: 46);

(7) complementarity determining regions of a heavy chain, CDR1 (SYWMH, SEQ ID No.: 49), CDR2 (EINPSNGRTNYNEKFKN, SEQ ID No.: 50), and CDR3 (RGTSGNYFDY, SEQ ID No.: 51), and complementarity determining regions of a light chain, CDR1 (KASQDINTYLS, SEQ ID No.: 52), CDR2 (RANRLVD, SEQ ID No.: 53), and CDR3 (LQYDEFPYT, SEQ ID No.: 54); and

(8) complementarity determining regions of a heavy chain, CDR1 (SYWMN, SEQ ID No.: 57), CDR2 (RIDPYDSEAHYNQKFKD, SEQ ID No.: 58), and CDR3 (IRYGTFDY, SEQ ID No.: 59), and complementarity determining regions of a light chain, CDR1 (KASQDINSYLS, SEQ ID No.: 60), CDR2 (RSNSLVD, SEQ ID No.: 61), and CDR3 (LQYDEFPYT, SEQ ID No.: 62).

2. The antibody or human-type antibody derivative according to claim 1, wherein the activation of T cells is selected from the group consisting of T cell proliferation, increased T cell cytotoxicity against cancer cells, and promotion of T cell cytokine secretion.

3. The antibody or human-type antibody derivative according to claim 1 or 2, wherein the human-type antibody derivative is selected from a human-type antibody variant selected from a humanized antibody, a chimeric antibody, a polyvalent antibody, and a multispecific antibody, or a functional fragment thereof.

4. The antibody or human-type antibody derivative according to any one of claims 1 to 3, wherein the functional fragment is F(ab')2.

5. The antibody or human-type antibody derivative according to any one of claims 1 to 4, wherein an amino acid sequence of a heavy chain variable region VH domain of the antibody or human-type antibody derivative is selected from (1) SEQ ID No.: 7, (2) SEQ ID No.: 15, (3) SEQ ID No.: 23, (4) SEQ ID No.: 31, (5) SEQ ID No.: 39, (6) SEQ ID No.: 47, (7) SEQ ID No.: 55, and (8) SEQ ID No. 63.

6. The antibody or human-type antibody derivative according to any one of claims 1 to 5, wherein an amino acid sequence of a light chain variable region VL domain of the antibody or human-type antibody derivative is selected from (1) SEQ ID No.: 8, (2) SEQ ID No.: 16, (3) SEQ ID No.: 24, (4) SEQ ID No.: 32, (5) SEQ ID No.: 40, (6) SEQ ID No.: 48, (7) SEQ ID No.: 56, and (8) SEQ ID No. 64.

7. The antibody or human-type antibody derivative according to any one of claims 1 to 6, wherein the antibody or human-type antibody derivative induces cytotoxicity against cancer cells but does not induce cytotoxicity against a normal cell.

8. The antibody or human-type antibody derivative according to any one of claims 1 to 7, wherein the cancer cell is selected from the group consisting of melanoma, breast cancer, lung cancer, and colorectal cancer.

9. The antibody or human-type antibody derivative according to any one of claims 1 to 8, wherein the antibody or human-type antibody derivative is bound to a drug to form an antibody drug conjugate (ADC).

10. A pharmaceutical composition for treating a cancer, comprising the antibody or human-type antibody derivative according to any one of claims 1 to 9.

11. The pharmaceutical composition according to claim 10, wherein the cancer is selected from the group consisting of melanoma, breast cancer, lung cancer, and colorectalcancer.

12. A method for measuring cytotoxicity against cancer cells, comprising:

a step of contacting in vitro cancer cells collected from a subject with the antibody or human-type antibody derivative according to any one of claims 1 to 9; and
a step of measuring whether or not AMP metabolism of the cancer cells is reduced, a step of measuring whether or not cell viability is reduced, or a step of measuring whether or not secretion of an immunostimulatory substance is enhanced, under a culture condition.

13. The method for measuring cytotoxicity against cancer cells according to claim 12, comprising measuring, in the presence of a peripheral blood lymphocyte of the same subject, whether or not cell viability of the cancer cell is reduced, or whether or not immune cells derived from the peripheral blood lymphocytes are activated.

14. The method for measuring cytotoxicity against cancer cells according to claim 12 or 13, comprising measuring, from in vitro AMP metabolism suppression or cytotoxicity against the cancer cells collected from the subject, an enhancement of cytotoxicity against the cancer cells when the antibody or human-type antibody derivative according to any one of claims 1 to 9 is administered to the subject.

15. The method for measuring cytotoxicity against cancer cells according to claim 12 or 13, comprising measuring, from in vitro enhancement of secretion of the immunostimulatory substance, an enhancement of cytotoxicity against the cancer cell when the antibody or human-type antibody derivative according to any one of claims 1 to 9 is administered to the subject.

16. A measurement kit comprising the antibody or the human-type antibody derivative according to any one of claims 1 to 9 for in vitro measuring AMP metabolism, cytotoxicity, secretion of an immunostimulatory substance, or activation of immune cells against the cancer cell collected from the subject, by the antibody or the human-type antibody derivative.

Figure 1

Figure 2

| | 10 | 20 | 30 | 40 | 50 | 60 |
|---|---|---|---|---|---|---|
| **Heavy chain** | QVQMKQSGPE | LVKPGASVKI | PCKASGYTFT | DCNMDWVKQS | HGESLEWIGD | INPNNGGTIY |
| | 70 | 80 | 90 | 100 | 110 | 120 |
| | NQKFKGKVTL | TIDKSSSTAY | MELRSLTSED | TAVYYCARTN | WDYAMDYWGQ | GTSVTVSS |

| | 10 | 20 | 30 | 40 | 50 | 60 |
|---|---|---|---|---|---|---|
| **Light chain** | DIQMTQSPSS | MYASLGERVT | ITCKASQDIN | SNLSWFQQKP | GKSPKTLIYR | ANRLIDGVPS |
| | 70 | 80 | 90 | 100 | 110 | 120 |
| | RFSGSGSGQD | YSLTISSLEY | EDMGIYYCLQ | YDVFPRTFGG | GTKLELK | |

Figure 3

002_1_c

002_2_c

002_3_c

002_4_c

002_5_c

002_6_c

———— Subject antibody    ▓▓▓▓ Isotype control

Figure 4

- 002_1_c
- 002_2_c
- 002_3_c
- 002_4_c
- 002_5_c
- 002_6_c
- Benchmark antibody
- Isotype control

Figure 5-1

```
002_6_c_HC    1  QVQMKQSGPELVKPGASVKIPCKASGYTFTDSNMDWVKQSHGESLEWIGDINPNNGGTIY  60
002_6_h1_HC   1  QVQLVQSGAEVKKPGASVKVSCKASGYTFTDSNMDWVRQAPGQRLEWMGDINPNNGGTIY  60
002_6_h2_HC   1  QVQMKQSGAEVKKPGASVKVSCKASGYTFTDSNMDWVRQAPGQRLEWMGDINPNNGGTIY  60
002_6_h3_HC   1  QVQLLQSAAEVRKPGASVKVSCKASGYTFTDSNMDWVRQAPGQGLEWMGDINPNNGGTIY  60
002_6_h4_HC   1  QVQMKQSAAEVRKPGASVKVSCKASGYTFTDSNMDWVRQAPGQGLEWMGDINPNNGGTIY  60
002_6_h5_HC   1  QVQLQESGAEVKKPGASVKVSCKASGYTFTDSNMDWVRQAPGQGLEWMGDINPNNGGTIY  60
002_6_h6_HC   1  QVQMKESGAEVKKPGASVKVSCKASGYTFTDSNMDWVRQAPGQGLEWMGDINPNNGGTIY  60
002_6_h7_HC   1  EVQLVESGAEVKKPGASVKVSCKASGYTFTDSNMDWVRQAPGQGLEWMGDINPNNGGTIY  60
002_6_h8_HC   1  EVQLVESGAEVKKPGASVKVSCKASGYTFTDSNMDWVRQAPGQGLEWMGDINPNNGGTIY  60


002_6_c_HC   61  NQKFKGKVTLTIDKSSSTAYMELRSLTSEDTAVYYCARTNWDYAMDYWGQGTSVTVSS  118
002_6_h1_HC  61  NQKFKGRVTITRDTSASTAYMELSSLRSEDTAVYYCARTNWDYAMDYWGQGTLVTVSS  118
002_6_h2_HC  61  NQKFKGRVTITIDTSASTAYMELSSLRSEDTAVYYCARTNWDYAMDYWGQGTLVTVSS  118
002_6_h3_HC  61  NQKFKGRVTMTTDTSRRTAYMELRSLRSDDTAVYYCARTNWDYAMDYWGQGTTVTVSS  118
002_6_h4_HC  61  NQKFKGRVTMTIDTSRSTAYMELRSLRSDDTAVYYCARTNWDYAMDYWGQGTTVTVSS  118
002_6_h5_HC  61  NQKFKGRVTMTRDTSTSTVYMELSSLRSEDTAVYYCARTNWDYAMDYWGQGTLVTVSS  118
002_6_h6_HC  61  NQKFKGRVTMTIDTSTSTVYMELSSLRSEDTAVYYCARTNWDYAMDYWGQGTLVTVSS  118
002_6_h7_HC  61  NQKFKGRVTMTRDTSINTAYMELSRLRSDDTAVYYCAMTNWDYAMDYWGQGTTVTVSS  118
002_6_h8_HC  61  NQKFKGRVTMTRDTSINTAYMELSRLRSDDTAVYYCAMTNWDYAMDYWGQGTTVTVSS  118
```

Figure 5-2

```
002_6_c_LC    1 DIQMTQSPSSMYASLGERVTITCKASQDINSQLSWFQQKPGKSPKTLIYRANRLIDGVPS 60

002_6_h1_LC   1 DIQMTQSPSSLSASVGDRVTITCKASQDINSQLSWYQQKPGKAPKLLIYRANRLIDGVPS 60

002_6_h2_LC   1 DIQMTQSPSSMSASVGDRVTITCKASQDINSQLSWYQQKPGKAPKTLIYRANRLIDGVPS 60

002_6_h3_LC   1 DIQMTQSPSFLSASVGDRVTITCKASQDINSQLSWYQQKPGKAPKLLIYRANRLIDGVPS 60

002_6_h4_LC   1 DIQMTQSPSFLSASVGDRVTITCKASQDINSQLSWYQQKPGKAPKTLIYRANRLIDGVPS 60

002_6_h5_LC   1 DIQVTQSPSFLSASVGDRVTITCKASQDINSQLSWYQQKPGKAPKLLIYRANRLIDGVPS 60

002_6_h6_LC   1 DIQVTQSPSFLSASVGDRVTITCKASQDINSQLSWYQQKPGKAPKTLIYRANRLIDGVPS 60

002_6_h7_LC   1 DIVMTQSPSSLSASVGDRVTITCKASQDINSQLSWYQQKPGKAPKLLIYRANRLIDGVPS 60

002_6_h8_LC   1 DIVMTQSPSSLSASVGDRVTITCKASQDINSQLSWYQQKPGKAPKLLIYRANRLIDGVPS 60


002_6_c_LC   61 RFSGSGSGQDYSLTISSLEYEDMGIYYCLQYDVFPRTFGGGTKLELK               107

002_6_h1_LC  61 RFSGSGSGTDFTLTISSLQPEDFATYYCQQYDVFPRTFGQGTKLEIK               107

002_6_h2_LC  61 RFSGSGSGTDFTLTISSLQYEDMATYYCLQYDVFPRTFGQGTKLEIK               107

002_6_h3_LC  61 RFSGSGSGTEFTLTISSLQPEDFATYYCQQYDVFPRTFGGGTKLEIK               107

002_6_h4_LC  61 RFSGSGSGTDFTLTISSLQYEDMATYYCLQYDVFPRTFGGGTKLEIK               107

002_6_h5_LC  61 RFSGSGSGTEFTLTISSLQPEDFATYYCQQYDVFPRTFGGGTKVEIK               107

002_6_h6_LC  61 RFSGSGSGTDFTLTISSLQYEDMATYYCLQYDVFPRTFGGGTKVEIK               107

002_6_h7_LC  61 RFSGSGSGTDFTLTISSLQPEDFATYYCQQYDVFPRTFGQGTKVEIK               107

002_6_h8_LC  61 RFSGSGSGTDFTLTISSLQPEDFATYYCQQYDVFPRTFGQGTKVEIK               107
```

Figure 6-1

Figure 6-2

Figure 7

Figure 8-1

MDA-MB-231

HEK293T
wild-type cell

HEK293T
CD73 forced
expression
cell

——— Subject antibody      Isotype control

Figure 8-2

Figure 9

Figure 10

Figure 11

**T cell division**

**IFN-gamma secretion**

Figure 12

Figure 13

Figure 14-1

```
003_m_HC     1 DVKLVESGGGLVQPGGSRKLSCAASGFTFSSFGMHWVRQAPEKGLEWVAYISSGSRTIYY  60

004_m_HC     1 QVQLQQPGAELVRPGTSVKLSCKASGYTFTGYWMNWLKQRPDQGLEWIGRIDPYDSETHY  60

005_m_HC     1 DVQLVESGPGLVAPSQSLSITCTVSGFSLTSYGVSWVRQPPGKGLEWLGVIWGDGST-NY  59

006_m_HC     1 QVQLQQPGAELVKPGVSVKLSCKASGYTFTSYWMHWVKQRPGQGPEWIGEINPSNARTNY  60

007_m_HC     1 QVQLQQPGAELVRPGASVKLSCKASGYTFTSYWMNWVKQRPEQGLEWIGKIDPYDSETHY  60

008_m_HC     1 QVQLKQSGAELVKPGASVKLSCKASGYTFTSYWMHWVKQRPGQGPEWIGEINPSNGRTNY  60

009_m_HC     1 QVQLQQSGAELVRPGASVKLSCKASGYTFTSYWMNWVKQRPEPGLEWIGRIDPYDSEAHY  60


003_m_HC    61 ADTVRGRFTISRDNSKNTLFLQLTSLRSEDTAIYYCASDFGSSSPNYFDY---WGQGTTV 117

004_m_HC    61 SQKFKDKAILTVHKSSSTAYIQLNSLTSEDSAVYYCTRSSPITTAPFDY----WGRGTTL 116

005_m_HC    60 HSALISRLSISKDNSKSQVFLKLNSLQTDDTATYYCAKTNIFYDYDWYLDV--WGAGTTV 117

006_m_HC    61 NENFKSKATLTVDKSSTAYMHFSRLTSEDSAVYYCARRGTSGNYFDF------WGQGTTV 115

007_m_HC    61 NQKFKDKAILTVDKSSSTAFMQLSSLTSEDSAVYYCARIRYGTFDY-------WGQGTTL 113

008_m_HC    61 NEKFKNRATLTVDKSSSTAYMQLSRLTSEDSAVYYCARRGTSGNYFDY-----WGQGTTL 115

009_m_HC    61 NQKFKDKAILTVDKSSSTAYMQLSSLTSEDSAVYYCTRIRYGTFDY-------WGQGTTL 113


003_m_HC   118 TVSS                                                         121

004_m_HC   117 TVSS                                                         120

005_m_HC   118 TVSS                                                         121

006_m_HC   116 TVSS                                                         119

007_m_HC   114 TVSS                                                         117

008_m_HC   116 TVSS                                                         119

009_m_HC   114 TVSS                                                         117
```

Figure 14-2

```
003_m_LC    1 DIVMTQSPASLAVSLGQRATISCRASESVDNYGISFMN---WFQQKPGQPPKLLIYAASN 57

004_m_LC    1 NIVLTQSPASLAVSLGQRATISCRASESVDYYGFSFMN---WFQLKPGQPPKLLIYAAST 57

005_m_LC    1 DIVMTQAPLSLPVSLGDQASISCRSSQSLVHSNGNTYLH--WYLQKPGQSPKLLIYKVSN 58

006_m_LC    1 QIVLTQSPAILSASPGEKVTMTCSASSSVSYMQ--------WFQQKPGASPKLWMYSISN 52

007_m_LC    1 DIVMTQSPSSMYASLGERVTITCKASQDINNYLS-------WFQQKPGKSPKTLIYRANI 53

008_m_LC    1 DIQMTQSPSSMYASLGERVTITCKASQDINTYLS-------WFQQKPGKSPKTLIYRANR 53

009_m_LC    1 DIVLTQSPSSMYASLGERVTITCKASQDINSYLS-------WFQQKPGKSPKTLIYRSNS 53


003_m_LC   58 QGSGVPARFSGSGSGTDFSLNIHPMEEDDTAMYFCQQSKEVPWTFGGGTRLEIK       111

004_m_LC   58 QGSGVPARFSGSGSGTDFSLNIHPMEEDDIAMYFCQQSKEVPYTFGGGTKLELK       111

005_m_LC   59 RFSGVPDRFSGSGSGTDFTLKISRVEAEDLGVYFCSHSTHVPWTFGGGTKLELK       112

006_m_LC   53 LAFGVPARFSGSGSGTSYSLTISSVKAEDAATYYCQQWSSSPLTFGAGTKLEIK       106

007_m_LC   54 LVDGVPSGFSGSGSGQDYSLTISSLEYEDMGIYYCLQYDEFPYTFGGGTRLEIK       107

008_m_LC   54 LVDGVPSRFSGSGSGEDYSLTISSLEYEDMGIYYCLQYDEFPYTFGGGTRLEIK       107

009_m_LC   54 LVDGVPSRFSGSGSGQDYSLTISSLEYEDMGSYYCLQYDEFPYTFGGGTRLEIK       107
```

Figure 15

Figure 16

Figure 17

Figure 18

Figure 19

Figure 20-1

Figure 20-2

Figure 21

Figure 22

Figure 23

Figure 24

|  | 002_6_h4 | 006_hKB | Benchmark antibody |
| --- | --- | --- | --- |
| 15 min | | | |
| 19 hr | | | |

Blue: DAPI staining (nuclear staining)
Red: Antibody distribution
Cell: MDA-MB-231

Figure 25

Figure 26

**MDA-MB-231**

Benchmark antibody
002_6_h4
006_hKB

**HT-144**

Benchmark antibody
002_6_h4
006_hKB

Figure 27

Tumor infiltrating T cell (analysis by each donors)

CD8 positive T cell

CD4 positive T cell

Figure 28-1

NCI-H292

NCI-H292 (100h)

Figure 28-2

Figure 29

Donor #5915

Donor #0030

Figure 30

| | Treatment | Dose (mg/kg) | Route | Frequency |
|---|---|---|---|---|
| ▲ | 002_6_h4 | 20 | IP | Q4Dx7 |
| ▽ | 002_6_h4 | 10 | | |
| ■ | 002_6_h4 | 5 | | |
| ⊝ | Isotype control | 20 | | |

Figure 31

**INTERNATIONAL SEARCH REPORT**

| International application No. |
| --- |
| PCT/JP2021/020384 |

**A. CLASSIFICATION OF SUBJECT MATTER**
Int.Cl. C07K16/30(2006.01)i, A61K39/395(2006.01)i, A61K45/00(2006.01)i,
A61K47/68(2017.01)i, A61P35/00(2006.01)i, A61P43/00(2006.01)i,
C07K16/46(2006.01)i, C12Q1/04(2006.01)i
FI: C07K16/30ZNA, C07K16/46, C12Q1/04, A61P35/00, A61K39/395T, A61K39/395P,
A61P43/00107, A61K47/68, A61P43/00111, A61K45/00
According to International Patent Classification (IPC) or to both national classification and IPC

**B. FIELDS SEARCHED**

Minimum documentation searched (classification system followed by classification symbols)
Int.Cl. C07K16/30, A61K39/395, A61K45/00, A61K47/68, A61P35/00, A61P43/00,
C07K16/46, C12Q1/04

Documentation searched other than minimum documentation to the extent that such documents are included in the fields searched
Published examined utility model applications of Japan     1922-1996
Published unexamined utility model applications of Japan   1971-2021
Registered utility model specifications of Japan           1996-2021
Published registered utility model applications of Japan   1994-2021

Electronic data base consulted during the international search (name of data base and, where practicable, search terms used)
JSTPlus/JMEDPlus/JST7580 (JDreamIII); CAplus/REGISTRY (STN)

**C. DOCUMENTS CONSIDERED TO BE RELEVANT**

| Category* | Citation of document, with indication, where appropriate, of the relevant passages | Relevant to claim No. |
| --- | --- | --- |
| X | JP 2018-501197 A (MEDIMMUNE LIMITED) 18 January 2018 (2018-01-18), claims, examples | 1-16 |
| X | JP 2015-143226 A (FUJITA HEALTH UNIVERSITY) 06 August 2015 (2015-08-06), claims, examples | 1-16 |
| X | JP 2019-503709 A (CORVUS PHARMACEUTICALS, INC. et al.) 14 February 2019 (2019-02-14), claims, examples | 1-16 |
| X | HAMMAMI, A. et al., Targeting the adenosine pathway for cancer immunotherapy, Seminars in Immunology, 2019, vol. 42, no. 101304, pp. 1-7, 2. Blocking the CD73-adenosine pathway in cancer therapy, table 1, 4. Clinical trials | 1-16 |

☐ Further documents are listed in the continuation of Box C.       ☒ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 28 July 2021 | 17 August 2021 |

| Name and mailing address of the ISA/ | Authorized officer |
| --- | --- |
| Japan Patent Office 3-4-3, Kasumigaseki, Chiyoda-ku, Tokyo 100-8915, Japan | |
| | Telephone No. |

Form PCT/ISA/210 (second sheet) (January 2015)

| JP 2018-501197 A | 18 January 2018 | US 2016/0194407 A1<br>claims, examples<br>EP 3218406 A1 |
| JP 2015-143226 A | 06 August 2015 | US 2009/0203538 A1<br>claims, examples<br>EP 2078732 A1 |
| JP 2019-503709 A | 14 February 2019 | US 2019/0077873 A1<br>claims, examples<br>EP 3387442 A1 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2018501197 A
- WO 2018501197 A

### Non-patent literature cited in the description

- *Oncoimmunology,* August 2016, vol. 5 (8), e1208875
- *Bioinformatics,* 01 February 2015, vol. 31 (3), 434-5
- *Clin. Microbiol. Rev.,* 1994, vol. 7, 277-289
- **LOTTA VON BOEHMER et al.** *Nature Protocols,* 2016, vol. 11, 1908-1923